# EUROPEAN PATENT SPECIFICATION

(11) **EP 1 526 839 B1**
(45) Date of publication and mention of the grant of the patent: **14.03.2007**
(21) Application number: 03760660.5
(22) Date of filing: 20.06.2003
(51) Int. Cl.: A61K 9/107, A61K 31/216, A61K 31/235, A61K 31/407, A61K 31/426, A61K 31/44, A61K 31/4164, A61K 31/4709

(54) **ORAL PHARMACEUTICAL FORMS OF LIQUID DRUGS HAVING IMPROVED BIOAVAILABILITY**
ORALE, PHARMAZEUTISCHE DARREICHUNGSFORMEN VON FLÜSSIGEN MEDIKAMENTEN MITVERBESSERTER BIOVERFÜGBARKEIT
FORMES PHARMACEUTIQUES ORALES DE MEDICAMENTS LIQUIDES A BIODISPONIBILITE AMELIOREE

(30) Priority: 25.06.2002 IT MI20021392
(43) Date of publication of application: 04.05.2005
(73) Proprietor: NicOx S.A., 06560 Sophia Antipolis - Valbonne (FR)
(72) Inventor: DEL SOLDATO, Piero, I-20052 Monza (IT); SANTUS, Giancarlo, I-20146 Milano (IT); MACELLONI, Cristina, I-20030 Bovisio Masciago (IT)
(74) Representative: Barchielli, Giovanna
(86) International application number: PCT/EP2003/006496
(87) International publication number: WO 2004/000273

(56) References cited:
- WO-A-00/61537
- WO-A-01/66087
- WO-A-01/66088

## Description

The present invention relates to new pharmaceutical compositions for the administration of liquid drugs in solid oral forms, said compositions comprising one or more active ingredients, one or more surface-active agents and optionally a co-surfactant and/or an absorption enhancer absorbed on a solid inert carrier.

It s well known in the art that it is difficult to orally administer drugs, which are liquid at room temperature. Generally, these drugs show a poor water solubility and therefore a limited absorption, resulting in a poor bioavailability together with an absorption characterized by a strong inter- and intra-subject variability. Therefore, it would be important to have at disposal compositions able to improve these characteristics that could seriously compromise the bioavailability as well as the therapeutic activity of said compounds.

Generally, oily drugs are formulated in soft or hard gelatine capsules which present technical problems relating to filling, losses etc. They can be also absorbed on inert carriers, but in this case even though the technological problems can be solved, it is impossible to improve the bioavailability.

In WO 01/66087 and WO 01/66088 pharmaceutical compositions for oral administration of a liquid active ingredient, for example a nitrooxyderivative of naproxen or other NSAIDs, are disclosed. Said compositions comprise, further to the active ingredient, one or more surfactants, optionally an oily or semi-solid fat or one or more short-chain alcohols. These compositions form an oil-in-water emulsion in situ upon contact with aqueous media such as gastrointestinal fluids.

In WO 95/08983 a self-emulsifying composition suitable for oral administration is disclosed, said composition forming a microemulsion in situ upon contact with biological fluids. The described composition comprises an active ingredient, a lipophilic phase consisting of a mixture of glycerides and fatty acids esters, a surface-active agent, a co-surfactant and a hydrophilic phase consisting of the gastrointestinal fluids.

In EP 274 870 a pharmaceutical composition containing a non-steroidal anti-inflammatory drug (NSAID) and a surfactant is described, said composition being able to form micelles containing said active ingredient upon oral administration

In WO 01/41737 an immediate-release solid oral pharmaceutical composition, comprising a solid carrier and a liquid drug or a solution of a poor soluble drug, is described.
It has been now surprisingly found that it is possible to improve the oral bioavailability of liquid drugs at room temperature, by formulating the solid drug in solid pharmaceutical compositions able to form emulsions in situ upon contact with the biological fluids and with the water used for ingesting the pharmaceutical form.
In particular, the present invention relates to the preparation of solid pharmaceutical compositions for oral administration consisting of an admixture absorbed in a solid inert carrier and an admixture comprising:
i) one or more liquid active ingredients and
ii) one or more surfactants and
iii) optionally a co-surfactant and/or
iv) optionally an absorption enhancer said composition forming an oil-in-water emulsion upon contact with aqueous media such as biological fluids and is characterized in that said admixture is absorbed in the inert carrier.
   Particularly preferred is a pharmaceutical composition according to claim 1 wherein the admixture absorbed in the inert carrier comprises:
   i) one or more liquid active ingredients;
   ii) one or more surfactants;
   iii) an absorption enhancer

For liquid active ingredient, a drug being liquid, generally oily, at room temperature is meant. Examples of drugs being oily liquids at room temperature are for example several nitrate esters of drugs such as the non-steroidal anti-inflammatory drugs (NSAIDs) described in EP 609415, EP 670825, EP 722434, EP 759899 and patent applications WO 00/51988, WO 00/61537, WO 00/61541 e WO 01/54691 in the name of applicant.

Examples of said nitrate esters are the following:
(S)-3-benzoyl-α-methylbenzeneacetic acid 3-(nitrooxy)propyl ester 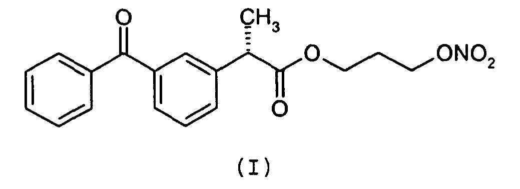
(S)-3-benzoyl-α-methylbenzeneacetic acid 4-(nitrooxymethyl)phenyl-methyl ester 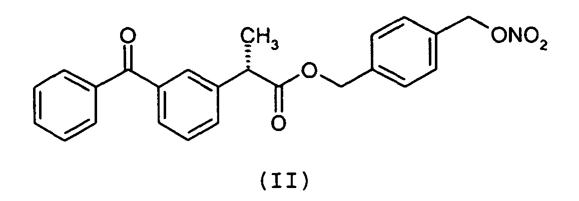
2-[(2,6-dichlorophenyl)amino]benzeneacetic acid 5-(nitrooxy)ethyl-oxyethyl ester 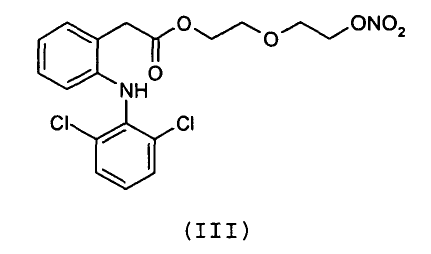
2-[(2,6-dichlorophenyl)amino]benzeneacetic acid 4-(nitrooxy)butyl ester (NO-Diclofenac) 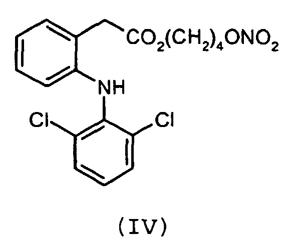
2-[(2,6-dichlorophenyl)amino]-benzeneacetic acid 3-(nitrooxy)propyl ester 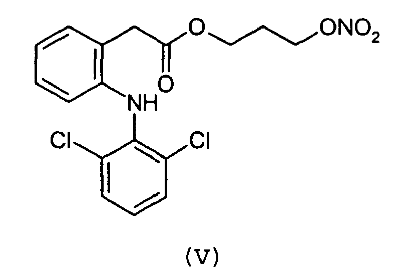
2-[(2,6-dichlorophenyl)amino]benzeneacetic acid 6-(nitrooxy)hexyl ester 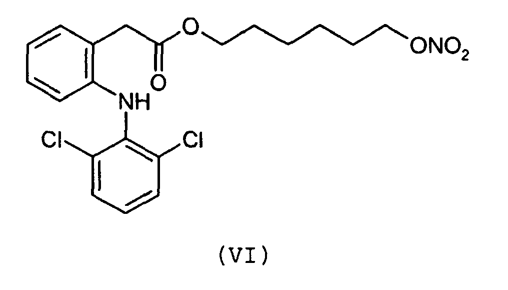
3-benzoyl-α-methylbenzeneacetic acid 4-(nitrooxy)butyl ester 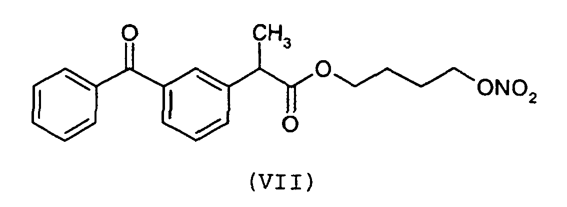
3-benzoyl-α-methylbenzeneacetic acid 6-(nitrooxy)hexyl ester 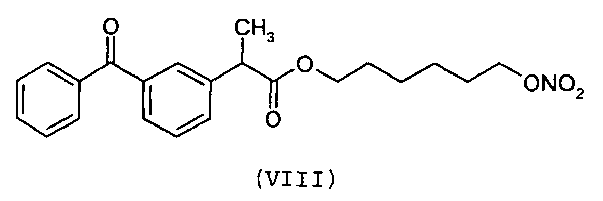
3-benzoyl-α-methylbenzeneacetic acid 5-(nitrooxy)ethyloxyethyl ester 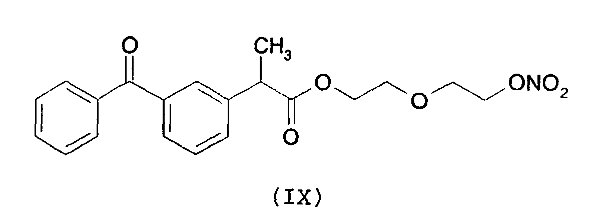
5-benzoyl-2,3-dihydro-1H-pyrrolizin-1-carboxylic acid 3-(nitrooxy)propyl ester 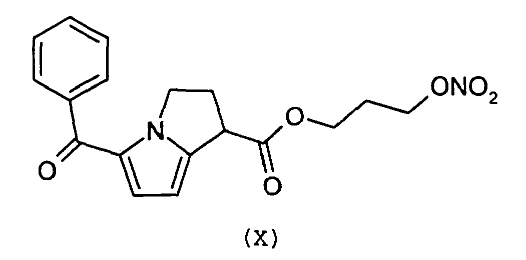
5-benzoyl-2,3-dihydro-1H-pyrrolizin-1-carboxylic acid 4-(nitrooxy)butyl ester 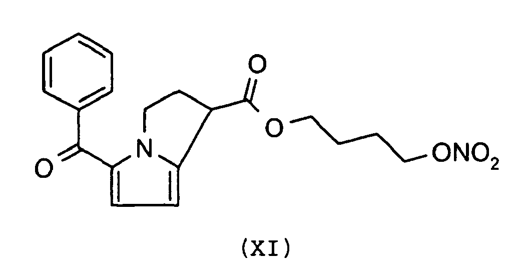
5-benzoyl-2,3-dihydro-1H-pyrrolizin-1-carboxylic acid 6-(nitrooxy)hexyl ester 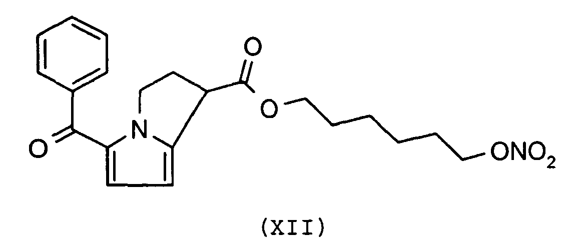
5-benzoyl-2,3-dihydro-1H-pyrrolizin-1-carboxylic acid 5-(nitrooxy)ethyl-oxyethyl ester 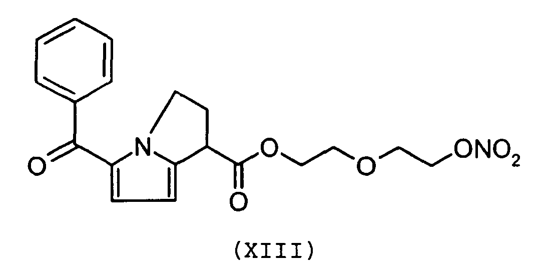
5-benzoyl-2,3-dihydro-1H-pyrrolizin-1-carboxylic acid 4-(nitrooxymethyl)-phenylmethyl ester 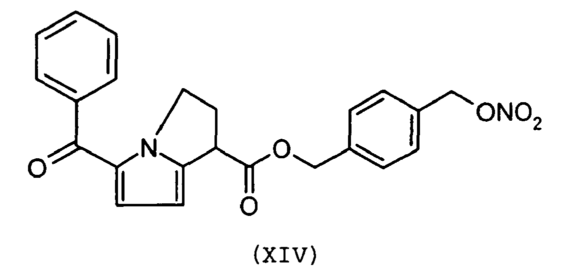
(S)-6-methoxy-α-methyl-2-naphthaleneacetic acid 6-(nitrooxy)hexyl ester 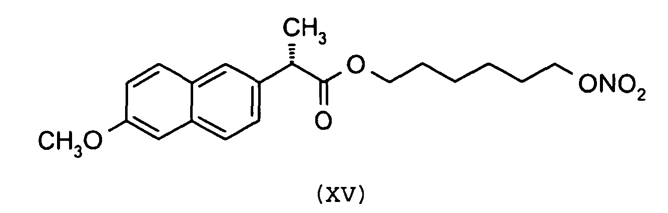
(S)-6-methoxy-α-methyl-2-naphthaleneacetic acid 5-(nitrooxy)ethyl-oxyethyl ester 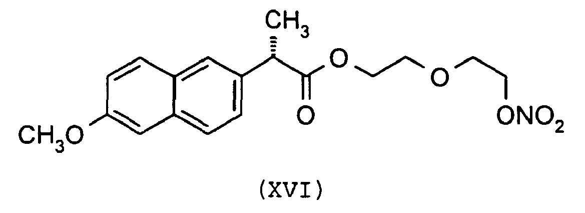
(S)-6-methoxy-α-methyl-2-naphthaleneacetic acid 4-nitrooxy-2-butenyl ester 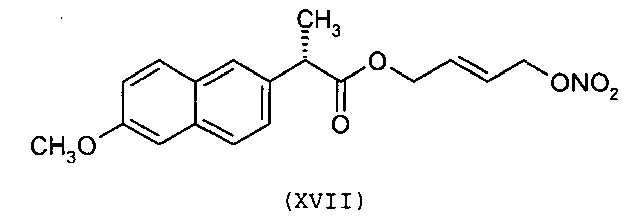
trans-3-[4-[α-methyl-4-(2-methylpropyl)benzene]acetyloxy]-3-methoxy-phenyl]-2-propenoic acid 4-(nitrooxy)butyl ester 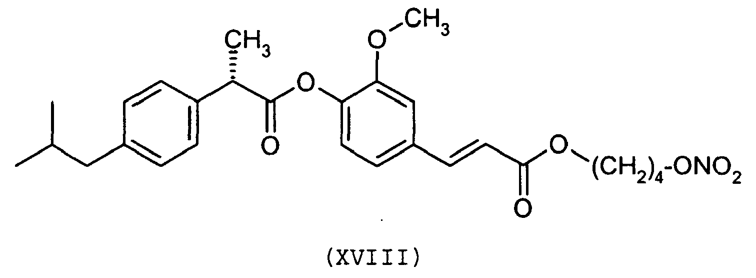
2-fluoro-α-methyl-[1,1'-biphenyl]-4-acetic acid 4-(nitrooxy)butyl ester (NO-Flurbiprofen) 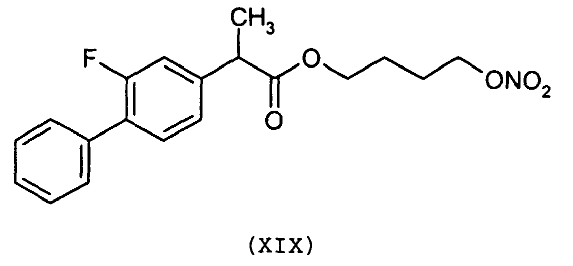
(S)-6-methoxy-α-methyl-2-naphthaleneacetic acid 4-(nitrooxy)butyl ester (NO-Naproxen) 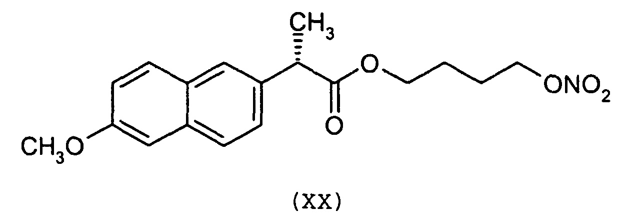
2-(acetyloxy)benzoic acid 4-(nitrooxy)butyl ester 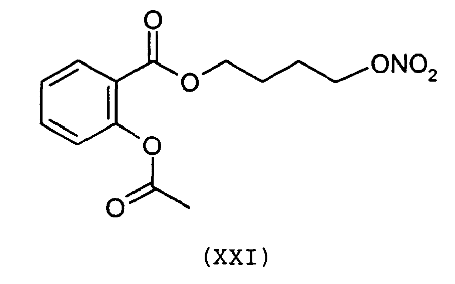
2-(acetyloxy)benzoic acid 5-(nitrooxy)ethyloxyethyl ester 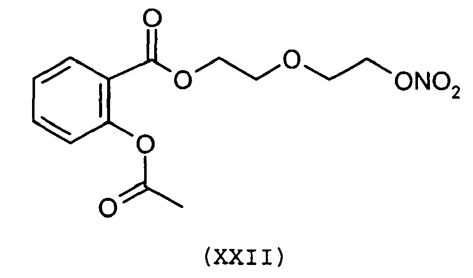
3-(6-methoxy-α-methyl-2-naphthaleneacetyl)-thiazolidine-4-carboxylic acid 4-(nitrooxy)-butyl ester 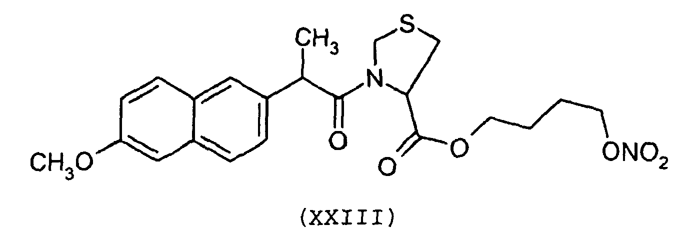
N-(2-nitrooxyethyl)-2-fluoro-α-methy[1,1'-biphenyl]-4-acetamide 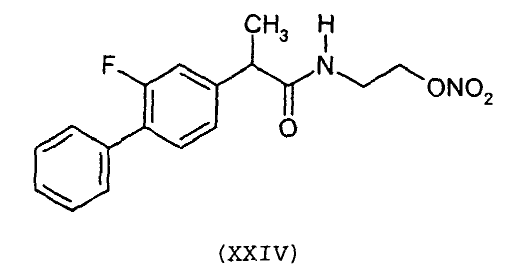
3-[2-fluoro-α-methyl-[1,1'-biphenyl]-4-acetyl]-thiazolidine-4-carboxylic acid 4-(nitrooxy)butyl ester 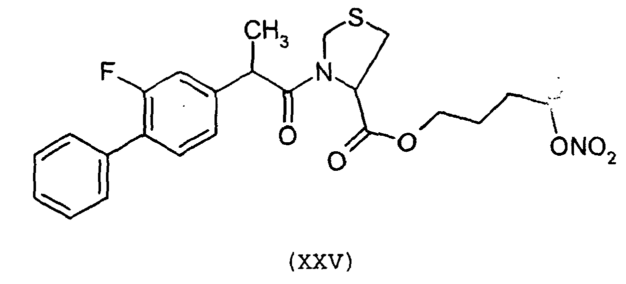
α-methyl-4-(2-methylpropyl)benzeneacetic acid 6-(nitrooxy)hexyl ester 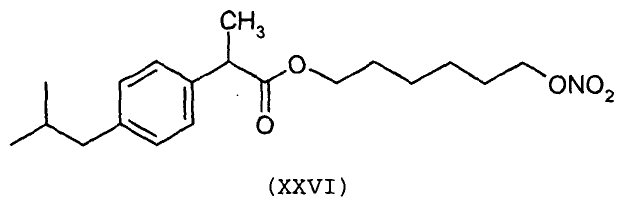
α-methyl-4-(2-methylpropyl)benzeneacetic acid 3-(nitrooxy)propyl ester 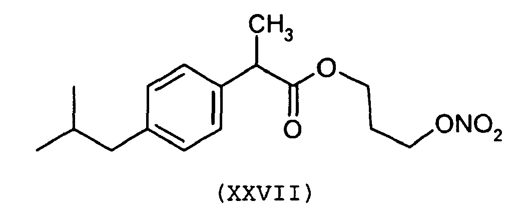
(S)-6-methoxy-α-methyl-2-naphthaleneacetic acid 1-nitrooxy-2-methyl-2-propyl ester 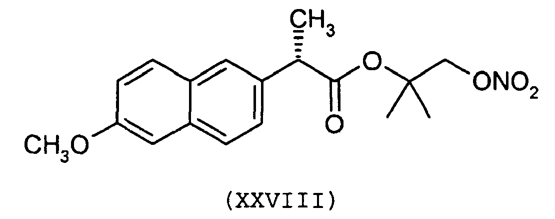
(S)-6-methoxy-α-methyl-2-naphthaleneacetic acid 10-(nitrooxy)decyl ester 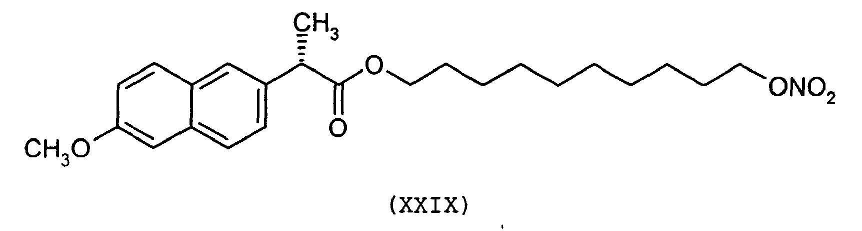
α-methyl-4-[(2-oxocyclopentyl)methyl]benzeneacetic acid 4-(nitrooxy)-butyl ester 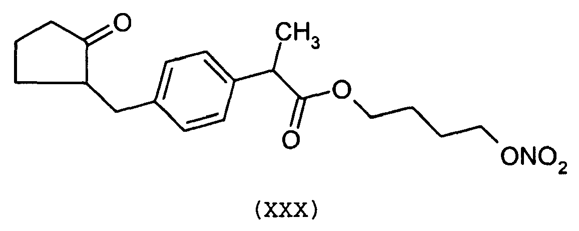
3-(6-methoxy-α-methyl-2-naphthaleneacetyl)-R(-)-2-oxothiazolidine-4-carboxylic acid 4-(nitrooxy)butyl ester 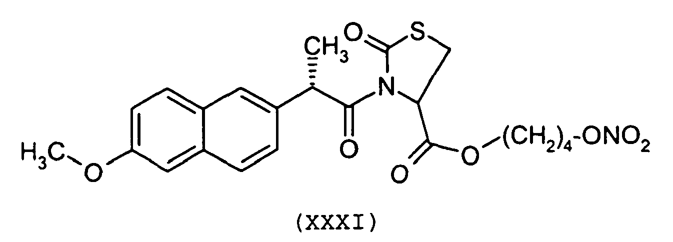
(S)-N-acetyl-[α-methyl-4-(2-methylpropyl)benzene-acetyl]-cysteine 4-(nitrooxy)butyl ester 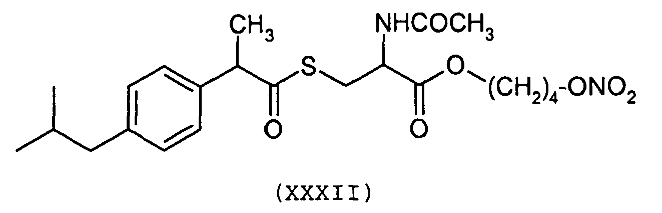
2-[2,6-dichlorophenyl)amino]benzeneacetic acid 2-(nitrooxy)ethyl ester 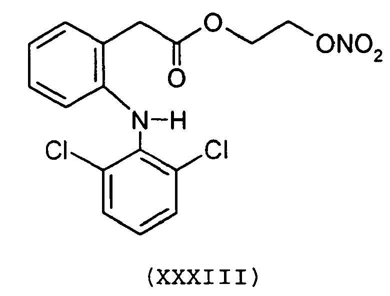
5-benzoyl-2,3-dihydro-1H-pyrrolizin-1-carboxylic acid 4-(nitrooxy-methyl)phenylmethyl ester 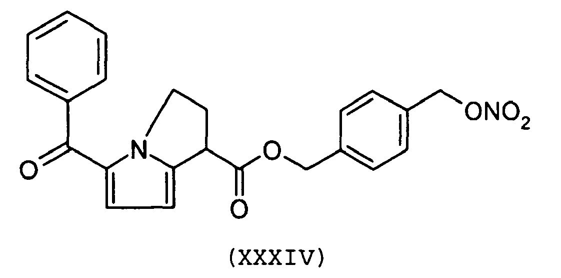
(S)-N-acetyl-[2-fluoro-α-methyl(1,1'-biphenyl)-4-acetyl]cysteine 4-(nitrooxy)butyl ester 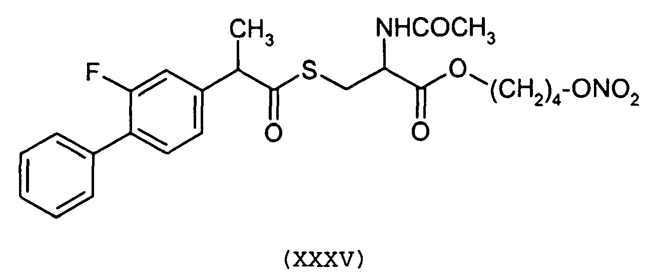
α-methyl-4-(2-methylpropyl)benzeneacetic acid 4-(nitrooxy)butyl ester 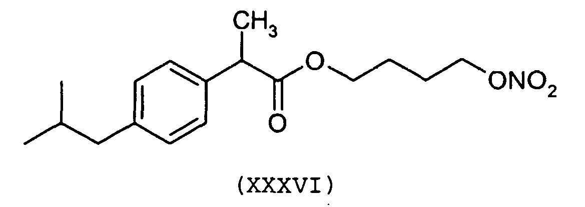
trans-3-[4-[2-fluoro-α-methyl(1,1'-biphenyl)-4-acetyloxy]-3-methoxy-phenyl]-2-propenoic acid 4-(nitrooxy)butyl ester 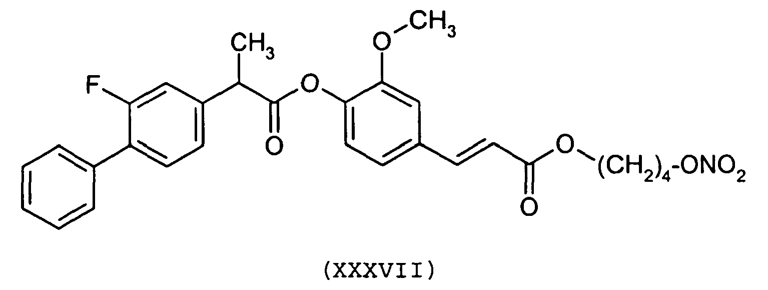
(S)-6-methoxy-α-methyl-2-naphthaleneacetic acid 4-(nitrooxy)-4-methylbutyl ester 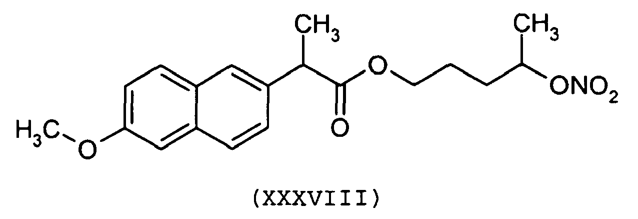
2-fluoro-α-methyl-[1,1'-biphenyl]-4-acetic acid 3-(nitrooxymethyl)phenyl ester 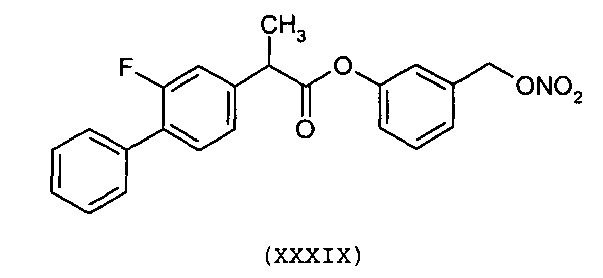
2-fluoro-α-methyl-[1,1'-biphenyl]-4-acetic acid 6-(nitrooxymethyl)-2-methylpyridyl ester 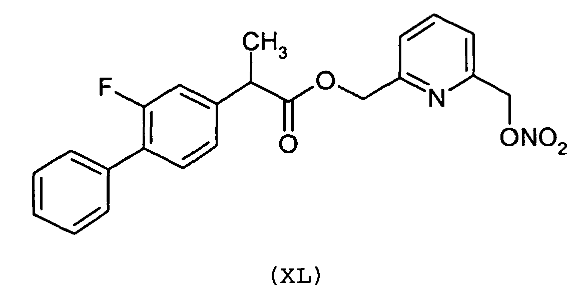
2-(acetyloxy)benzoic acid 3-(nitrooxymethyl)-methylphenyl ester 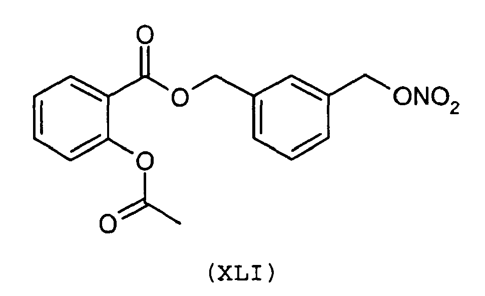
2-fluoro-α-methyl-[1,1'-biphenyl]-4-acetic acid 3-(nitrooxy)propyl ester 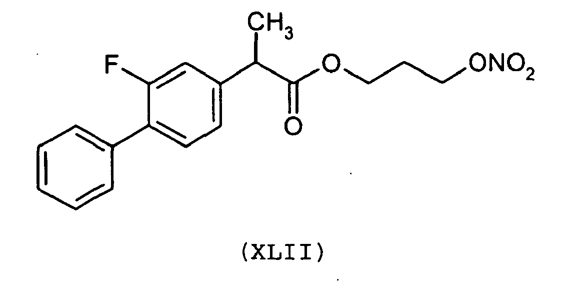
4-(nitrooxy)butanoic acid 2-methyl-5-nitroimidazole-1-ethyl ester 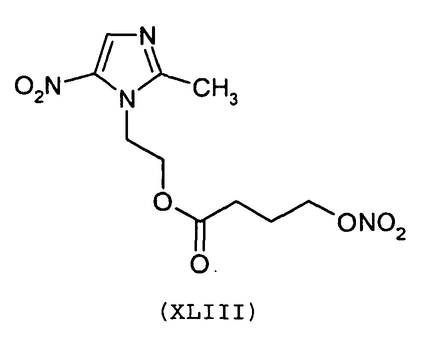
1-ethyl-6,8-difluoro-1,4-dihydro-7-(3-methyl-1-piperazinyl)-4-oxo-3-quinolinecarboxylic acid 4-(nitrooxy)butyl ester 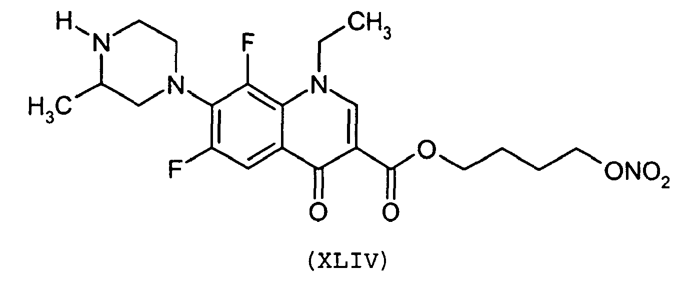
Norfloxacine 4-(nitrooxy)butyl ester

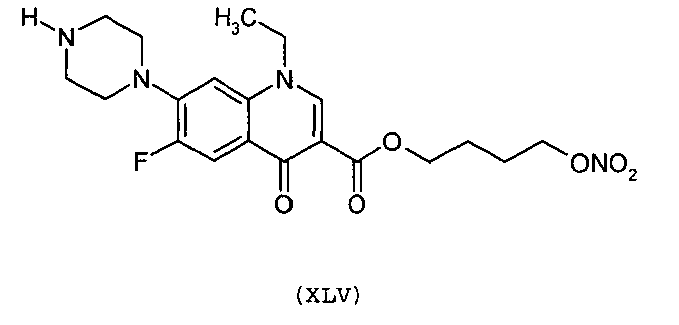

Further examples of liquid drugs are nicotine, nitroglycerin, valproic acid, benzonatate, clofibrate, clorfeniramine, clorfenoxamine, clorfentermine and clorpromazine and liquid vitamins.

The compositions of the invention are able to form an emulsion, upon ingestion of the pharmaceutical form by a patient, having reduced droplet size. The average droplet size of the emulsion is of from 0.1 and 50 microns and preferably is less than 5 micron.

The emulsion droplet size is measured by simulating the formation of an emulsion by adding in a beaker 50 ml of a 0.1N HCl aqueous solution and 100 mg of the composition under examination. The time required for the mixture to form an emulsion, can vary from 20 seconds to 10 minutes depending on the composition. The average droplet size of the emulsion was then determined by employing the light scattering technique or electronic microscopy.

Examples of surfactants that can be employed are anionic, non-ionic and cationic surfactants. Examples thereof may include, but are not limited to, alkaline soaps, such as sodium and potassium stearate, organic amines soaps, sulphuric esters, such as sodium lauryl sulphate, monolauryl glycerosulphuric acid sodium salt, alkyl aryl sulfonates, esters and ethers of polyethylene glycols, polysorbates, benzalkonium chloride, cetyltrimethylammonium bromide, cetrimide, particularly the commercially available products Arlacel, Tween, Capmul, Cremophor, Labrafac, Labrafil, Labrasol, etc. In a few cases it can be useful to add also co-surfactants, that is when a well definite HLB (hydrophilic-lipophilic balance) is requested. Preferred co-surfactants are straight or branched chain alcohols, preferably C₁-C₆ alcohols, such as ethyl alcohol, propyl alcohol, isopropyl alcohol, butyl alcohol, isobutyl alcohol, and polyols such as glycerol, ethylene glycol, propylene glycol, isopropylene glycol, butylene glycol, isobutylene glycol.

In order to improve the absorption, an absorption enhancer can be added to the active ingredient, dissolved or suspended in the surface-active agent and optionally in the co-surfactant. Many substances possess said activity and among these the following can be mentioned: polysorbates, sorbitan esters, sodium dioctyl sulfosuccinate, ethoxydiglycol, ethoxylated nonyl phenols, polyethylene lauryl ether, phospholipid derivatives, fatty acid esters, biliary acid derivatives, aprotic solvents such as dimethyl sulfoxide, dimethylformamide, dimethylacetamide and 2-pyrrolidone.

The active ingredient, surfactants and absorption enhancer admixture is allowed to absorb on an inert carrier in such a ratio to obtain a powder having good technological characteristics as far as for example free-flowing is concerned. For the absorption of said mixture generally granulators, kneaders or mixers normally used in the pharmaceutical field can be employed. Generally the mixture/solid carrier ratio may vary from 1:20 to 10:1 even though the preferred ratio is from 1:2 to 2:1.

As solid carrier any non toxic pharmaceutical compound may be used, including for example clays such as bentonite, kaolin, silica derivatives such as Aerosil, Cabosil, cellulose derivatives such as Avicel, silicates such as magnesium trisilicate, talc, hydroxides such as magnesium and aluminium hydroxide, starches, sugars and cyclodextrins. Silica is the preferred absorber.
The ratio by weight of active ingredient:surfactant may vary from 1:0.1 to 1:10, preferably of from 1:0.3 to 1:3. The ratio by weight of co-surfactant:surfactant may vary from 1:0.1 to 1:5, preferably of from 1:0.1 to 1:5.
The ratio by weight of absorption enhancer:surfactant may vary from 1:0.1 to 1:10, preferably of from 1:0.3 to 1:3.
The ratio by weight of admixture : solid carrier may vary from 1:20 to 10:1, preferably of from 1:2 to 2:1.

The resulting product is a free-flowing powder that can be employed in several pharmaceutical forms in the form for example of sachet), tablets (chewing, effervescent or quick dissolution tablets), controlled release capsules or tablets so as to have the active ingredient release in particular areas of the gastrointestinal tract; for this purpose, the coating will be gastroresistant or specifically directed into gut areas, for example colon.

Depending on the pharmaceutical form type, it is possible to use suitable excipients for having the desired formulation. Thus in the case of sachets, sugars, suspending agents, flavourings and sweeteners can be employed, whereas for tablets and capsules, diluents, disintegrants and lubricants can be used. Examples for these materials can be found in Remington's Pharmaceutical Sciences, 17th Edition, Mack Publishing Company, Easton, Pa., 1985.

### EXAMPLES

### Example 1

Preparation of 2-[(2,6-dichlorophenyl)amino]-benzeneacetic acid 4-(nitrooxy)butyl ester (NO-diclofenac; formula (IV)) absobed on colloidal silica

| | |
|---|---|
| Compound of formula (IV) | 100 g |
| Cremophor EL | 50 g |
| Phospholipon 80 H | 50 g |
| Aerosil 200 | 100 g |
| Explotab | 100 g |

Cremophor EL and compound of formula IV were added in a suitable vessel and mixed to homogeneity. In the same time Aerosil 200, Phospfolipon 80 H and Explotab were mixed separately. The powder mixture was slowly introduced in a mixer under stirring until complete absorption of the components was achieved. Emulsion average droplet size: 2,2 micron (minimum 0,27, maximum 13,3).

### Example 2

Preparation of a pharmaceutical powder form (sachet) for oral use employing the active ingredient mixture of Example 1

| | |
|---|---|
| Mixture of Example 1 | 400 g |
| Orange aroma powder | 150 g |
| Lemmon aroma | 50 g |
| Saccharin sodium | 10 g |
| Saccharose | 2390 g |

For preparing sachets, NO-diclofenac absorbed as described in Example 1 was mixed adding orange and lemon flavour as well as saccharin sodium and saccharose. A cube mixer was used with stirring at 9 rpm for 15 minutes. The mixture was distributed in sachets each weighing 3,0 g.

### Dissolution test

On the mixture obtained as described in Example 2, a dissolution test was carried out in 0.1N HCl at 37°C with a rotation speed of 50 rpm. The dissolution results are listed in Table 1.

**TABLE 1**

| | **NO-diclofenac absorbed on Aerosil 200 (without forming an emulsion)** | **Composition of the invention (example 2)** |
|---|---|---|
| *Time* | % *dissolved* | % *dissolved* |
| 0 | 0 | 0 |
| 15 | 3,4 | 88,7 |
| 30 | 4,8 | 90,2 |
| 60 | 5,7 | 93,2 |

### Example 3

Preparation of 2-fluoro-α-methyl(1,1'-biphenyl)-4-acetic acid 4-(nitrooxy)butyl ester (NO-flurbiprofen; formula (XIX)) absorbed on colloidal silica

| | |
|---|---|
| NO-flurbiprofen | 406 g |
| Cremophor EL | 106 g |
| Aerosil 200 | 300 g |
| Explotab | 200 g |

A suitable vessel was charged with NO-flurbiprofen and Cremophor EL and the mixture was stirred until a homogenous product was obtained. Separately, Aerosil 200 was mixed with Explotab and the whole was added to the previous mixture to give a homogenous mixture that was poured on a 0,85 mm sieve.
Average emulsion droplet size: 1.5 micron (minimum 0,20; maximum 12,8).

### Example 3.1

Preparation of a pharmaceutical powder form for oral use (sachets) employing the active ingredient mixture obtained in example 3

| | |
|---|---|
| Mixture of example 3 | 1000 g |
| Saccharin sodium | 20 g |
| Orange aroma | 300 g |
| Saccharose | 4674 g |

For preparing 3 g sachets, each containing 200 mg of active ingredient, 1000 g of the mixture obtained as previously described in example 3 were mixed with saccharin sodium, orange aroma and saccharose.

### Example 3.2

Preparation of tablets employing the mixture of example 3.

| | |
|---|---|
| Mixture of example 3 | 500 g |
| PVP K30 | 20 g |
| Avicel pH 102 | 277 g |

PVP K 30 was dissolved in 300 g water and the solution was used to wet the mixture of example 3 in a Erweka mixer. The product thus obtained was poured on a 2 mm sieve and then it was dried in an oven at 40°C for 3 hours. Afterwards, it was poured on a 1 mm sieve in a floating granulator and Avicel was added under stirring in a V mixer for 15 minutes. The product was compressed to the theoretical weight of 800 mg with a 18x10 mm oblong punch. Tablets having the following characteristics were obtained:
Title of a.i. NO-flurbiprofen: 201.3 mg/cpr
Hardness: 4 Kp
Friability: < 0.1%
Disgregation time: 4 min

### Example 4

Preparation of a solid pharmaceutical form (granulate) using (S)-6-methoxy-α-methyl-2-naphthaleneacetic acid 4-(nitrooxy)butyl ester (NO-Naproxen; compound of formula (XX))

| | |
|---|---|
| NO-Naproxen | 100 mg |
| Tween 80 | 50 mg |
| Phospholipon 80 H | 50 mg |
| Aerosil 200 | 100 mg |
| Explotab | 100 mg |

100 mg of Phospholipon 80H were dispersed in 2,5 ml water by heating at 85°C. The dispersion of Phospholipon 80 H was added under stirring to a mixture of NO-Naproxen and Tween 80. After adding Phospholipon, Aerosil and Explotab were added under stirring. A granulate was obtained and dried in an oven. The granulate was sieved through a 600 µm sieve. By dispersing 400 mg of this granulate in 20 ml water, an emulsion having an average droplet size of 2.2 micron was obtained (minimum 0.27; maximum 13.3).

### Example 5

Preparation of coated tablets employing the tablets obtained as described in example 3.2

| | |
|---|---|
| NO-Flurbiprofen tablets of ex. 3.2 | 800 g |
| Methocel E15 | 150 g |
| Titanium dioxide | 20 g |
| Talc | 20 g |
| PEG 600 | 30 g |
| 96% alcohol | 1600 g |

Methocel E 15 and PEG 6000 were dissolved in a suitable vessel and then talc and titanium dioxide were dispersed therein. The tablets prepared as described in example 3.2 were charged in a Pellegrini vessel and the tablet coating was performed with the film forming suspension according to the following parameters:
Air entry: 60°C (300 mc³/h)
Suction: 0,4 mc³/h
Drum rotation: 4 r/m
Film forming solution range: 30 ml/min

### Example 6

Preparation of gastroresistant coated tablets employing the tablets obtained as described in example 3.2

| | |
|---|---|
| Tablets prepared according to example 3.2 | 19 kg |
| Eudragit E 30 D | 0.49 kg |
| Talc | 0.19 kg |
| Triethyl citrate | 0.05 kg |
| Titaniun dioxide | 0.05 kg |
| Silicon antifoam | 0.005 kg |

Eudragit L30D was poured in 1.1 kg water under stirring to avoid foaming. 6,5 g NaOH were added and stirring was continued for further 30 minutes. A latex was obtained that was sieved through a 0,25 mm mesh sieve. Triethyl citrate, talc and antifoam agent were added, then the suspension was homogenized together with the Eudragit suspension. The tablets prepared according to example 3.2 were introduced into a vessel and sprayed with the mixture obtained as mentioned above, by employing a peristaltic pump and a Graco atomizer gun. The mixture was sprayed with a pressure of 1,5 bar and at a rate of 40 g/minute with an air capacity of 7 m³/minute at 55°C. The tablets temperature was maintained at 34°C.

### Example 7

In man evaluation of pharmacokinetic and pharmacodynamic parameters of the oral NO-diclofenac formulation described in example 2 (sachets).

Six healthy fastened patients were administered with 75, 100 and 150 mg NO-Diclofenac sachets formulated as described in example 2.

In order to evaluate the pharmacokinetic parameters, blood samples were taken at 0.25, 0.5, 1, 2, 3, 4, 5, 6, 8, 10, 12, 16, 24 and 32 hours after administration of the pharmaceutical formulation. The active ingredient NO-diclofenac and its metabolites diclofenac and the hydroxyderivative 4-OH-diclofenac were dosed in plasma by a LC/MS/MS method, previously validated. NO-diclofenac was not found in the samples at any dosage. The pharmacokinetic parameters of plasma levels obtained for diclofenac (D) and the 4-hydroxydiclofenac (40H-Diclofenac, 40H-D) are reported in Table 2.

The inhibition of COX-1/COX-2, in blood samples taken at 0.5, 1, 3, 6, 10, 24 and 32 hours after administration, was also evaluated in the same patients. The obtained results are listed in Table 3.

**TABLE 2**

| | **Sachets 100 mg** | |
|---|---|---|
| | D | *4OH-D* |
| Cmax (ng/mL) | 415.7 | 281 |
| Tₘₐₓ (h) | 0.55 | 1.4 |
| t1/2 (h) | 6.85 | 11.3 |
| AUC₍₀₋ₜ₎ | 1097.1 | 2446.2 |
| AUC_{(0-∞)} | 1168.5 | 2909.0 |
| MRT (h) | 7.31 | 16.1 |

**TABLE 3: COX 1 and COX 2 inhibition**

| | | | | | | |
|---|---|---|---|---|---|---|
| | **Predose** | | **1 h** | | **10 h** | |
| | | % | | % | | % |
| | *ng*/*ml* | *Inhibition* | *ng*/*ml* | *Inhibition* | *ng*/*ml* | *Inhibition* |
| COX 1 | 24.95 | 0 | 5.28 | -60 % | 14.03 | -19 % |
| (TBX2) | | | | | | |
| COX 2 | 55.29 | 0 | 29.55 | -75% | 10.55 | -108% |
| (PGE2) | | | | | | |

The results obtained both as pharmacokinetics and as pharmacodynamics confirm that the NO-diclofenac formulation described in example 2 has a good bioavailability in terms of plasmatic levels of diclofenac and of anti-inflammatory activity measured according to the ciclooxygenase 1 and 2 inhibition.

### Example 8

Comparison of NO-flurbiprofen bioavailability (Formula XIX) formulated in usual gelatine capsules vs sachets and tablets.

### 8.1: Pharmaceutical forms

### 8.1.A) Usual tablets

| | |
|---|---|
| NO-flurbiprofen | 100 mg |
| Mais starch | 300 mg |
| Avicel | 40 mg |
| Talc | 20 mg |
| Colloidal silica | 5 mg |
| Carboxymethylcellulose | 40 mg |
| Magnesium stearate | 10 mg |

The active ingredient was absorbed on starch and silica without surfactants and absorption enhancers. After absorption, the granulate was mixed with talc, magnesium stearate and carboxymethylcellulose and filled in hard gelatine capsules.

### 8.1.B) Sachets

Sachets have been prepared as described in example 3.1

### 8.1.C) Tablets

Tablets have been prepared as described in example 3.2
The bioavailability study has been performed on 12 healthy subjects. The subjects were administered each at three different times and in a randomized way with two 100 mg capsules, 200 mg caps and 200 mg tablets containing each NO-flurbiprofen.

Blood samples were taken after each administration at the here below listed times: 0.25, 0.50, 1, 2, 3, 4, 5, 6, 8, 10, 12, 16, and 24 hours. Flurbiprofen concentration in every plasmatic sample was determined by a LC/MS/MS method.

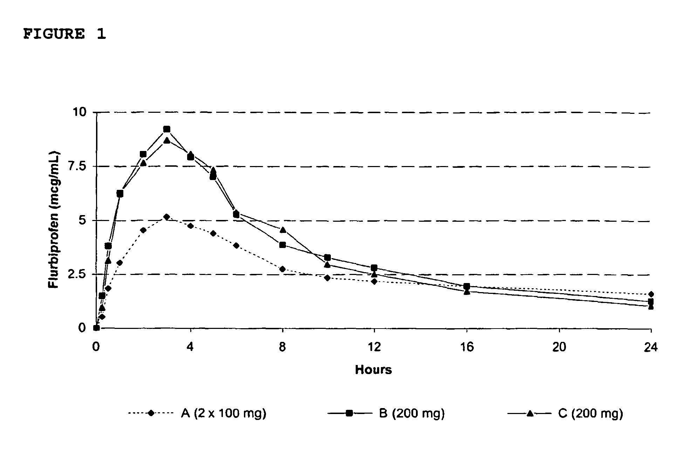

The obtained results are reported in Figure 1, and the pharmacokinetic parameters are presented in Table 4.

The obtained results show that both sachets and tablets are non-bioequivalent in comparison with usual capsules, as they give a better absorption both in terms of Cₘₐₓ and in terms of AUC.

**TABLE 4**

| | | | |
|---|---|---|---|
| | **Formulation 8.1.A** | **Formulation 8.1. B** | **Formulation 8.1.C** |
| | **(2x100 mg capsules)** | **(200 mg sachets)** | **(200 mg tablets)** |
| Cₘₐₓ | 5.8 | 9.7 | 9.2 |
| (µg/mL) | | | |
| Tₘₐₓ (h) | 3 | 3 | 3 |
| t1/2 (h) | 21.2 | 8.7 | 10.2 |
| AUC₍₀₋ₜ₎ | 62.7 | 86.3 | 83.2 |

## Claims

1. A pharmaceutical composition for oral administration consisting of a solid inert carrier and an admixture comprising:
i) one or more liquid active ingredients
ii) one or more surfactants
iii) optionally a co-surfactant and/or
iv) optionally an absorption enhancer
said composition forming an oil-in-water emulsion upon contact with aqueous media and is **characterized in that** said admixture is absorbed in the inert carrier.

2. A pharmaceutical composition according to claim 1 wherein the admixture absorbed in the inert carrier comprises:
i) one or more liquid active ingredients;
ii) one or more surfactants;
iii) an absorption enhancer

3. A pharmaceutical composition according to claim 1 or 2 wherein said composition forms an oil-in-water emulsion with an average droplet size of from 0.05 micron to 50 micron upon contact with aqueous media such as biological fluids.

4. A pharmaceutical composition according to claim 1 or 2 wherein said composition forms an oil-in-water emulsion with an average droplet size of less than 5 micron upon contact with aqueous media such as biological fluids.

5. A pharmaceutical composition according to claims 1 to 4, wherein the liquid active ingredient is a NO-releasing non-steroidal anti-inflammatory drug.

6. A pharmaceutical composition according to claim 5, wherein the NO-releasing non-steroidal anti-inflammatory drug is selected from the group consisting of:
(S)-3-benzoyl-α-methylbenzeneacetic acid 3-(nitrooxy) propyl ester 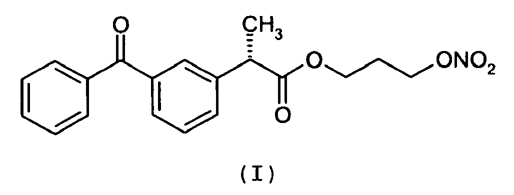
(S)-3-benzoyl-α-methylbenzeneacetic acid 4-(nitrooxymethyl)-phenylmethyl ester 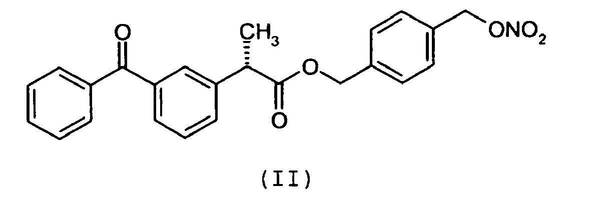
2-[(2,6-dichlorophenyl)amino]benzeneacetic acid 5-(nitrooxy)ethyl-oxyethyl ester 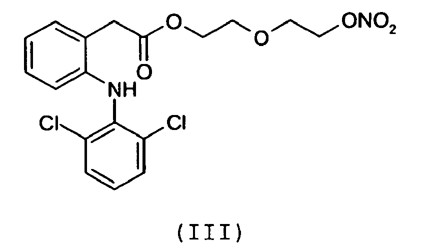
2-[(2,6-dichlorophenyl)amino]benzeneacetic acid 4(nitrooxy)butyl ester (NO-Diclofenac) 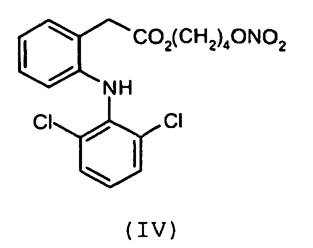
2-[(2,6-dichlorophenyl)amino]-benzeneacetic acid 3-(nitrooxy)propyl ester 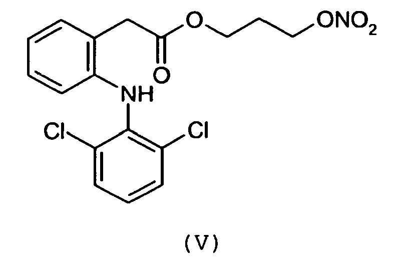
2-[(2,6-dichlorophenl)amino]benzeneacetic acid 6-(nitrooxy)hexyl ester 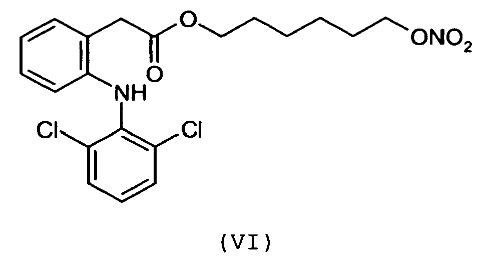
3-benzoyl-α-methylbenzeneacetic acid 4-(nitrooxy)butyl ester 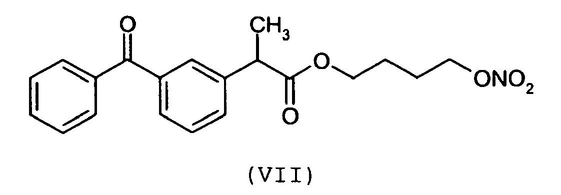
3-benzoyl-α-methylbenzeneacetic acid 6-(nitrooxy)hexyl ester 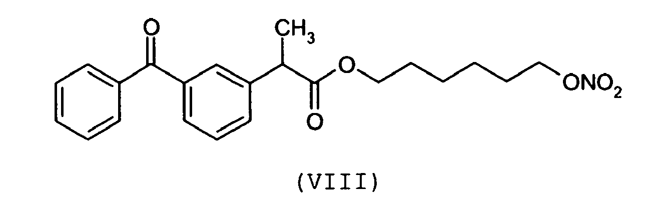
3-benzoyl-α-methylbenzeneacetic acid 5-(nitrooxy)ethyloxyethyl ester 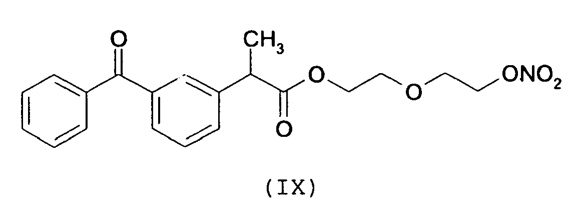
5-benzoyl-2,3-dihydro-1H-pyrrolizin-1-carboxylic acid 3-(nitrooxy)propyl ester 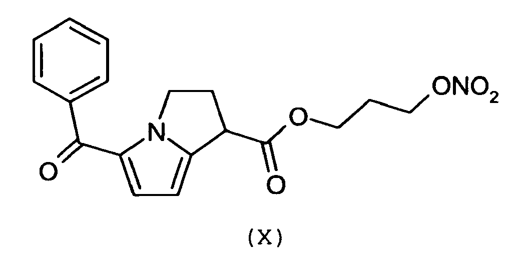
5-benzoyl-2,3-dihydro-1H-pyrrolizin-1-carboxylic acid 4-(nitrooxy)butyl ester 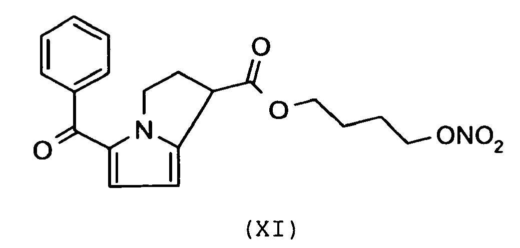
5-benzoyl-2,3-dihydro-1H-pyrrolizin-1-carboxylic acid 6-(nitrooxy)hexyl ester 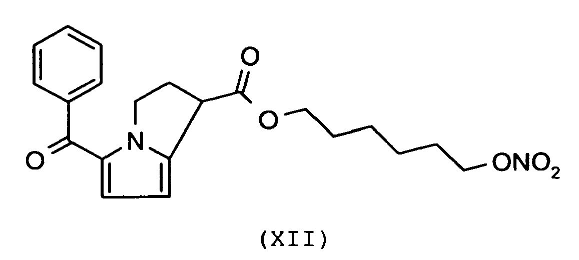
5-benzoyl-2,3-dihydro-1H-pyrrolizin-1-carboxylic acid 5-(nitrooxy)-ethyloxyethyl ester 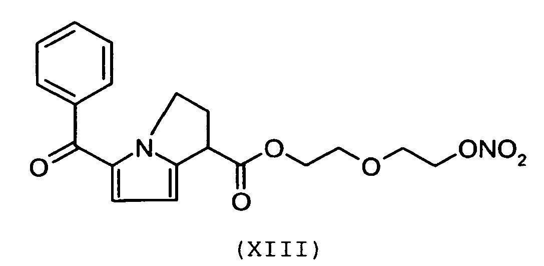
5-benzoyl-2,3-dihydro-1H-pyrrolizin-1-carboxylic acid 4-(nitrooxymethyl)-phenylmethyl ester 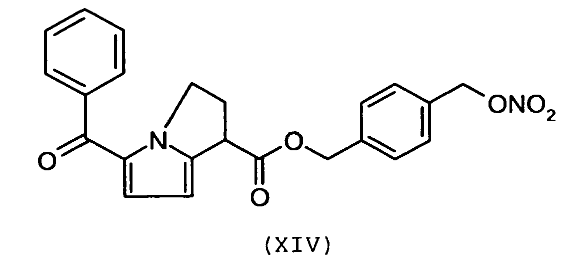
(S)-6-methoxy-α-methyl-2-naphtaleneacetic acid 6-(nitroxy)hexyl ester 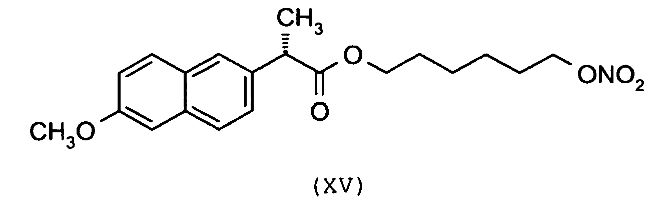
(S)-6-methoxy-α-methyl-2-naphtaleneacetic acid 5-(nitrooxy)ethyl-oxyethyl ester 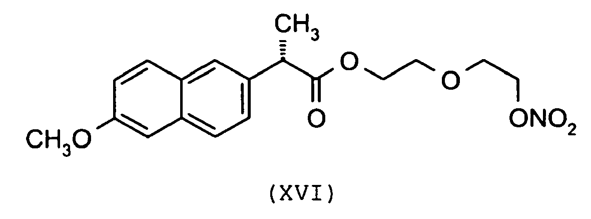
(S)-6-methoxy-α-methyl-2-naphtaleneacetic acid 4-nitrooxy-2-butenyl ester 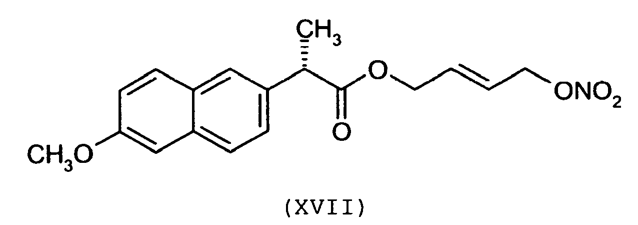
trans-3-[4-[α-methyl-4-(2-methylpropyl)benzene] acetyloxy]-3-methoxy-phenyl]-2-propenoic acid 4-(nitrooxy)butyl ester 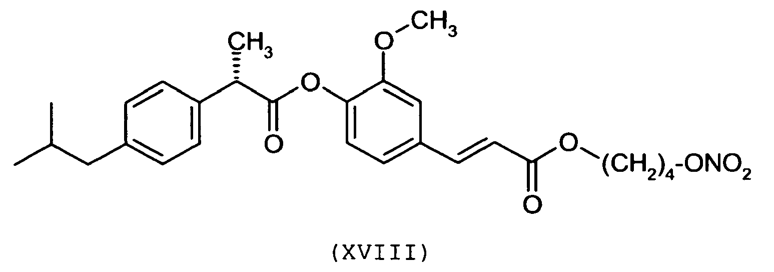
2-fluoro-α-methyl[1,1'-biphenyl]-4-acetic acid 4-(nitrooxy)butyl ester (NO-Flurbiprofen) 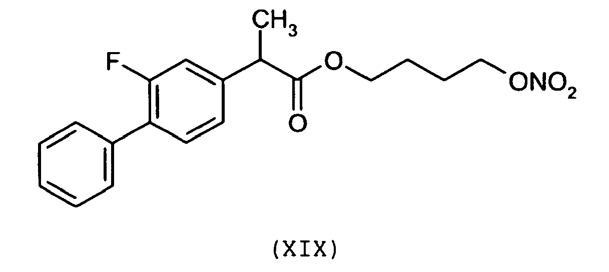
(S)-6-methoxy-α-methyl-2-naphtaleneacetic acid 4-(nitrooxy)butyl ester (NO-Naproxen) 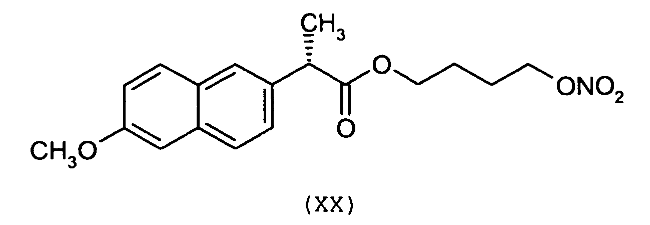
2-(acetyloxy)benzoic acid 4-(nitrooxy)butyl ester 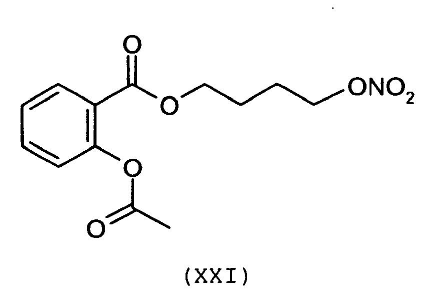
2-(acetyloxy)benzoic acid 5-(nitrooxy)ethyloxyethyl ester 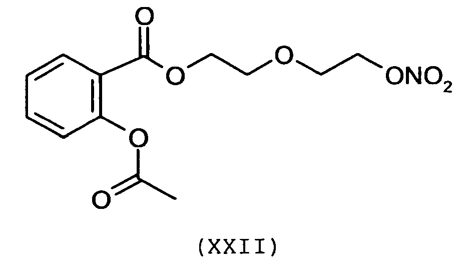
3-(6-methoxy-α-methyl-2-naphtalenacetyl)-thiazolidin-4-carboxylic acid 4-(nitrooxy)butyl ester 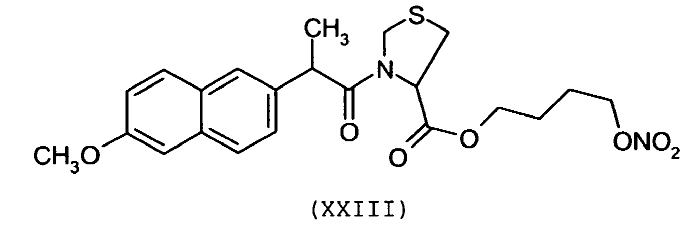
N-(2-nitrooxyethyl)-2-fluoro-α-methyl[1,1'-biphenyl]-4-acetamide 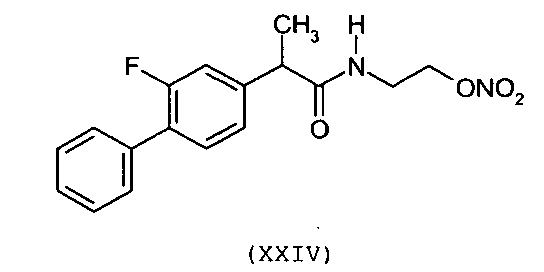
3-[2-fluoro-α-methyl-[1,1'-biphenyl]-4-acetyl]-thiazolidin-4-carboxylic acid 4-(nitrooxy)butyl ester 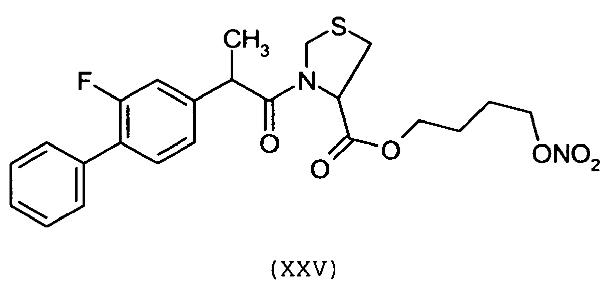
α-methyl-4-(2-methylpropyl)benzeneacetic acid 6-(nitrooxy)hexyl ester 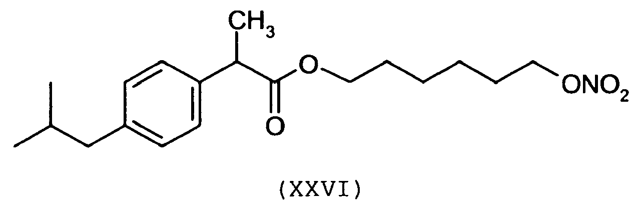
α-methyl-4-(2-methylpropyl)benzeneacetic acid 3-(nitrooxy)propyl ester 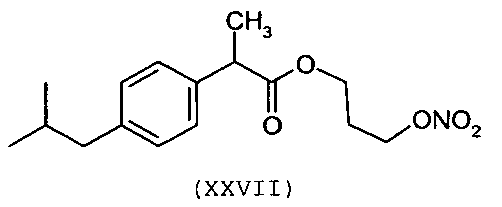
(S)-6-methoxy-α-methyl-2-naptaleneacetic acid 1-nitrooxy-2-metyl-2-propyl ester 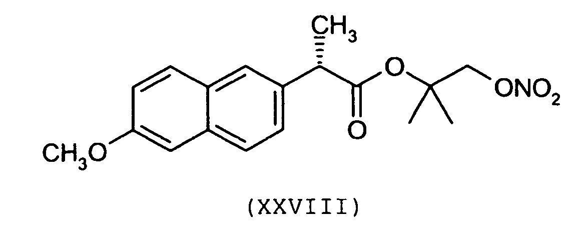
(S)-6-methoxy-α-methyl-2-naphtaleneacetic acid 10-(nitrooxy)decyl ester 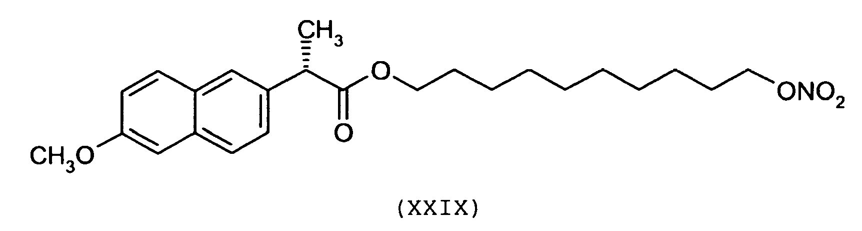
α-methyl-4-[(2-oxocyclopentyl)methyl]benzeneacetic acid 4-(nitrooxy)butyl ester 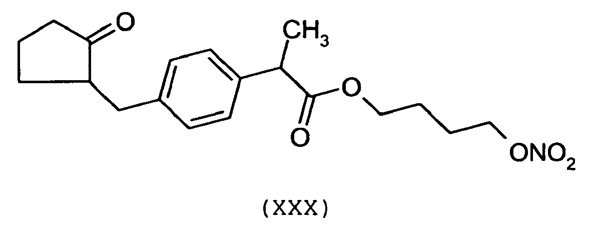
3-(6-methoxy-α-methyl-2-naphtalenacetyl)-R(-)-2-oxothiazolidin-4-carboxylic acid 4-(nitrooxy)butyl ester 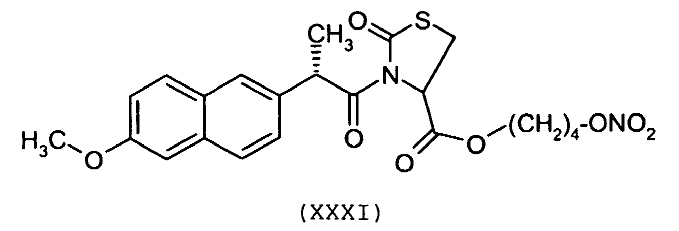
(S)-N-acetyl-[α-methyl-4-(2-methylpropyl)benzeneacetyl]-cysteine 4-(nitrooxy)butyl ester 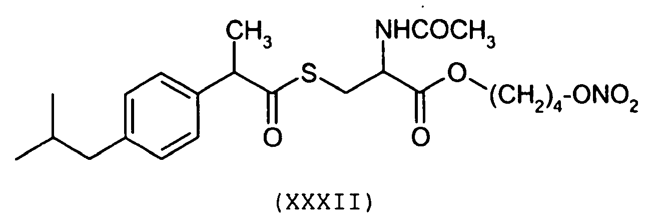
2-[2,6-dichlorophenyl)amino]benzeneacetic acid 2-(nitrooxy)etyl ester 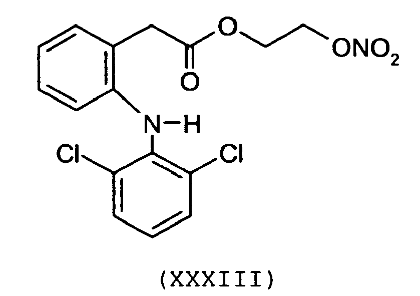
5-benzoyl-2,3-dihydro-1H-pyrrolizin-1-carboxylic acid 4-(nitrooxymethyl)-phenylmethyl ester 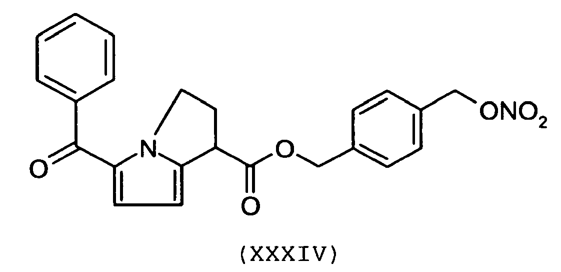
(S)-N-acetyl-[2-fluoro-α-methyl(1,1'-biphenyl)-4-acetyl]cysteine 4-(nitrooxy)butyl ester 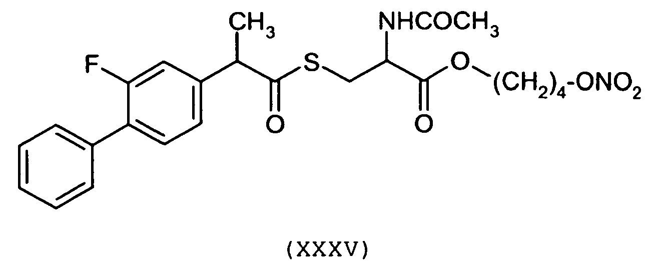
α-methyl-4-(2-methylpropyl)benzeneacetic acid 4-(nitrooxy)butyl ester 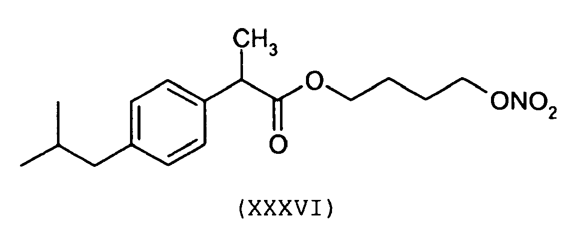
trans-3-[4-[2-fluoro-α-metyl(1,1'-biphenyl)-4-acetyloxy]-3-methoxy-phenyl]-2-propenoic acid 4-(nitrooxy)butyl ester 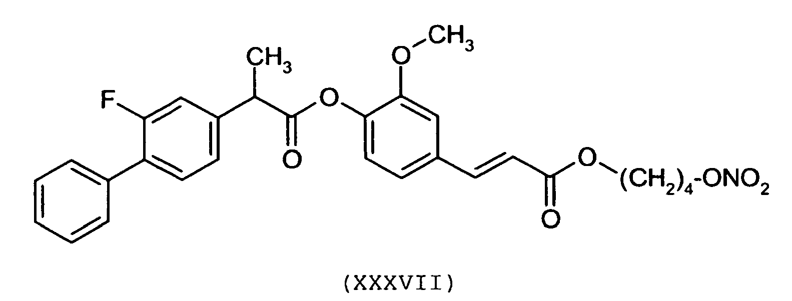
(S)-6- methoxy-α-methyl-2-naphtaleneacetic acid 4-(nitrooxy)-4-methylbutyl ester 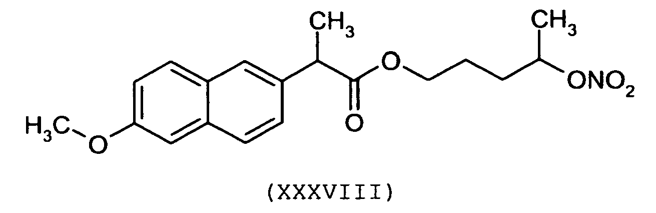
2-fluoro-α-methyl- [1,1'-biphenyl]-4-acetic acid 3-(nitrooxymethyl)phenyl ester 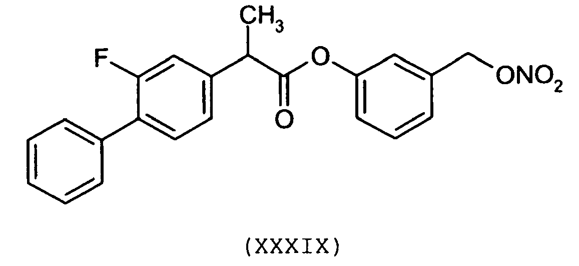
2-fluoro-α-methyl-[1,1′-biphenyl]-4-acetic acid 6-(nitrooxymethyl)-2-methylpyridyl ester 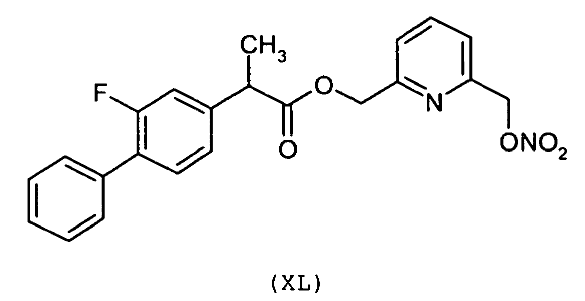
2-(acetyloxy)benzoic acid 3-(nitrooxymethyl)- methylphenyl ester 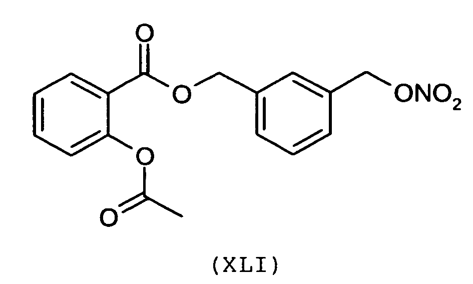
2-fluoro-α-methyl[1,1'-biphenyl]-4-acetic acid 3-(nitrooxy)propyl ester

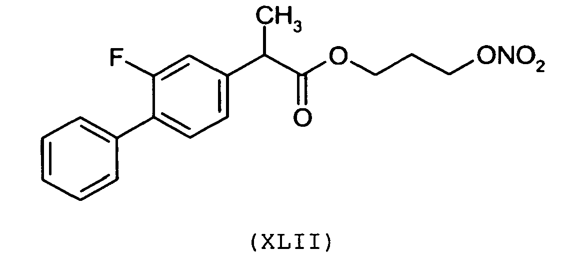

7. A pharmaceutical composition according to claims 1 to 4, wherein the liquid active ingredient is selected from
4-(nitrooxy)butanoic acid 2-methyl-5-nitroimidazole-1-ethyl ester 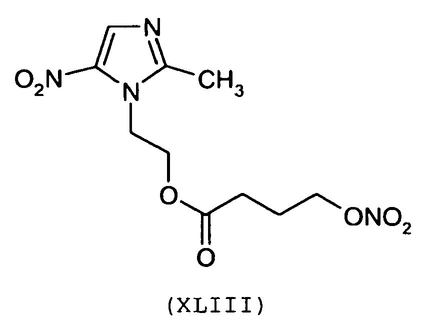 1-ethyl-6,8-difluoro-1,4-dihydro-7-(3-methyl-1-piperazinyl)-4-oxo-3-quinolinecarboxylic acid 4-(nitrooxy)butyl ester 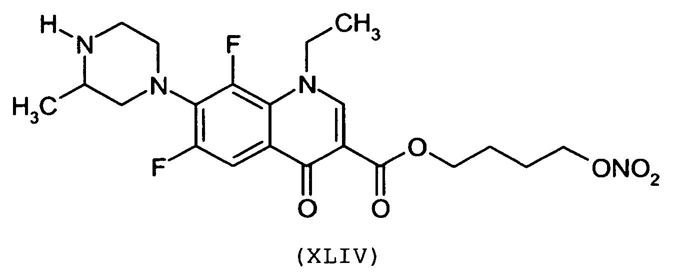 Norfloxacine 4-(nitrooxy)butyl ester

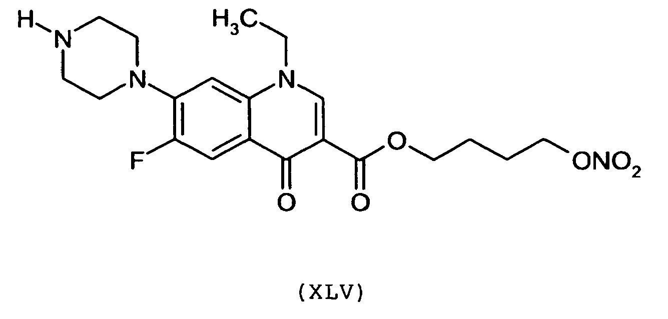

8. A pharmaceutical composition according to claims 1-4, wherein the liquid active ingredient is selected from the group consisting of nicotine, nitroglycerin, valproic acid, benzonatate, clofibrate, clorfeniramine, clorfenoxamine, clorfentermina and clorpromazine, liquid vitamins and mixtures thereof.

9. A pharmaceutical composition according to claim 1-8, wherein the surfactant is selected from cationic, anionic and non ionic surfactant such as alkaline soaps, organic amines soaps, sulphuric esters, alkyl aryl sulfonate, polyethylene glycol esters and ethers, polysorbates.

10. A pharmaceutical composition according to claim 9 wherein the surfactant is selected from the group consisting of sodium stearate, potassium stearate, sodium lauryl sulfate, sodium monolauryl glycerosulfate, benzalkonium chloride, cetyltrimethylammonium bromide, cetrimide, Arlacel, Tween, Capmul, Cremophor, Labrafac, Labrafil and Labrasol or mixtures thereof.

11. A pharmaceutical composition according to anyone of the preceding claims, wherein the co-surfactant is selected from straight or branched chain alcohols, preferably C₁-C₆ alcohols, such as ethyl alcohol, propyl alcohol, isopropyl alcohol, butyl alcohol, isobutyl alcohol, and polyols such as glycerol, ethylene glycol, propylene glycol, isopropylene glycol, butylene glycol, isobutylene glycol.

12. A pharmaceutical composition according to anyone of the preceding claims, wherein the absorption enhancer is selected from polysorbates, sorbitan esters, dioctyl sodium sulfosuccinate, ethoxy diglycol, ethoxylated nonyl phenols, polyethylene laurylether, phospholipid derivatives, fatty acids esters, biliary acids derivatives, aprotic solvents such as dimethyl sulfoxide, dimethylformamide, dimethylacetamide and 2-pyrrolidone.

13. A pharmaceutical composition according to anyone of the preceding claims, wherein the inert solid carrier is selected from the group consisting of clays such bentonite, kaolin, silica derivatives such as Aerosil, Carbosil, cellulose derivatives such as Avicel, silicates such as magnesium trisilicate, talc, earth-alkaline metal hydroxides such as magnesium and aluminium hydroxide, starch, sugars and cyclodextrines.

14. A pharmaceutical composition according to claim 10 wherein the inert solid carrier is silica.

15. A pharmaceutical composition according to anyone of the preceding claims, wherein the ratio of active ingredient:surfactant is of from 1:0.1 to 1:10.

16. A pharmaceutical composition according to anyone of the preceding claims, wherein the ratio of co-surfactant:surfactant is of from 1:0.1 to 1:5.

17. A pharmaceutical composition according to anyone of the preceding claims, wherein the ratio of absorption enhancer:surfactant is of from 1:0.1 to 1:10.

18. A pharmaceutical composition according to anyone of the preceding claims, wherein the ratio of admixture : solid carrier is of from 1:20 to 10:1, preferably of 1:2 to 2:1.

19. A pharmaceutical composition according to anyone of the preceding claims in form of tablets, coated tablets, sachets and capsules.

## Patentansprüche

1. Pharmazeutische Zusammensetzung zur oralen Verabreichung, bestehend aus einem festen inerten Träger und einem Zusatz, umfassend:
(i) einen oder mehrere flüssige Wirkstoffe,
(ii) ein order mehrere Tenside,
(iii) gegebenenfalls ein Co-Tensid und/oder
(iv) gegebenenfalls einen Absorptionsverstärker
wobei diese Zusammensetzung bei Kontakt mit einem wässrigen Medium eine Öl-in-Wasser-Emulsion bildet und **dadurch gekennzeichnet ist, dass** der Zusatz in dem inerten Träger absorbiert ist.

2. Pharmazeutische Zusammensetzung gemäss Anspruch 1, wobei der in dem inerten Träger absorbierte Zusatz umfasst:
(i) einen oder mehrere flüssige Wirkstoffe,
(ii) ein oder mehrere Tenside,
(iii) einen Absorptionsverstärker.

3. Pharmazeutische Zusammensetzung gemäss Anspruch 1 oder 2, wobei diese Zusammensetzung bei Kontakt mit einem wässrigen Medium, wie biologischen Fluiden, eine Öl-in-Wasser-Emulsion mit einer mittleren Tröpfchengrösse von 0,05 bis 50 µm bildet.

4. Pharmazeutische Zusammensetzung gemäss Anspruch 1 oder 2, wobei diese Zusammensetzung bei Kontakt mit einem wässrigen Medium, wie biologischen Fluiden, eine Öl-in-Wasser-Emulsion mit einer mittleren Tröpfchengrösse von weniger als 5 µm bildet.

5. Pharmazeutische Zusammensetzung gemäss Ansprüchen 1 bis 4, wobei der flüssige Wirkstoff ein NO-freisetzender, nicht-steroidaler Entzündungshemmer ist.

6. Pharmazeutische Zusammensetzung gemäss Anspruch 5, wobei der NO-freisetzende, nicht-steroidale Entzündungshemmer ausgewählt ist aus der Gruppe, bestehend aus:
(S)-3-Benzoyl-α-methylbenzolessigsäure-3-(nitrooxy)-propylester 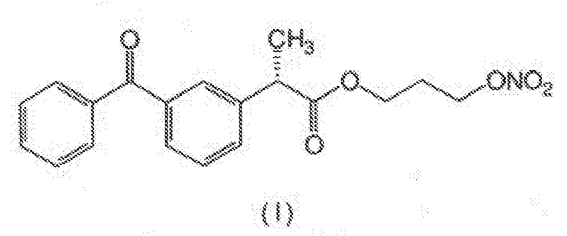
(S)-3-Benzoyl-α-methylbenzolessigsäure-4-(nitrooxymethyl)-phenylmetylester 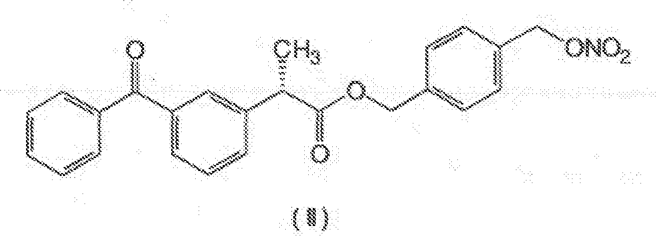
2-[(2,6-Dichlorphenyl)amino]benzolessigsäure-5-(nitrooxy)ethyl-oxyethylester 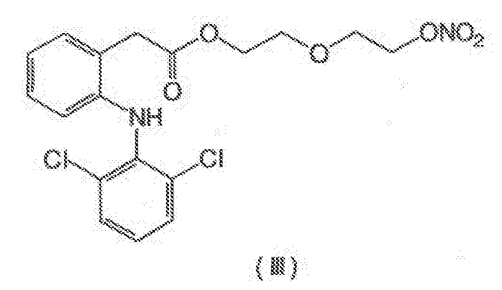
2-[(2,6-Dichlorphenyl)amino]benzolessigsäure-4-(nitrooxy)butylester (NO-Diclofenac) 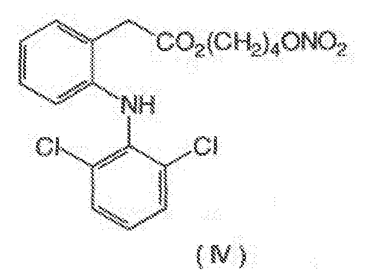
2-[(2,6-Dichlorphenyl)amino]-benzolessigsäure-3-(nitrooxy)propylester 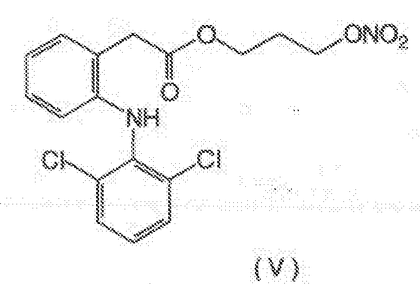
2-[(2,6-Dichlorphenyl)amino]benzolessigsäure-6-(nitrooxy)hexylester 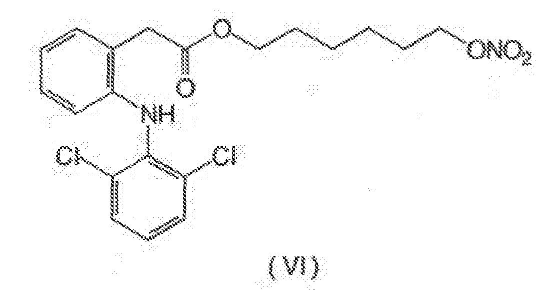
3-Benzoyl-α-methylbenzolessigsäure-4-(nitrooxy)-butylester 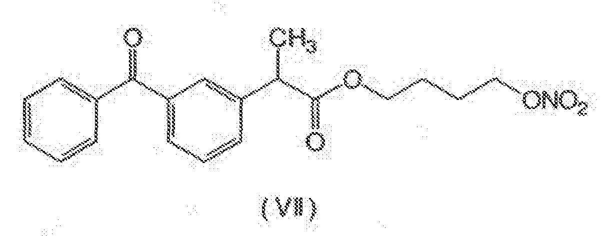
3-Benzoyl-α-methylbenzolessigäure-6-(nitrooxy)-hexylester 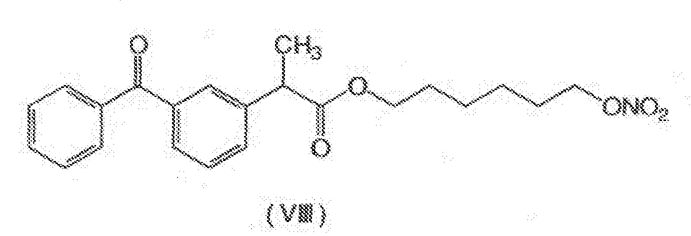
3-Benzoyl-α-methylbenzolessigäure-5-(nitrooxy)-ethyloxyethylester 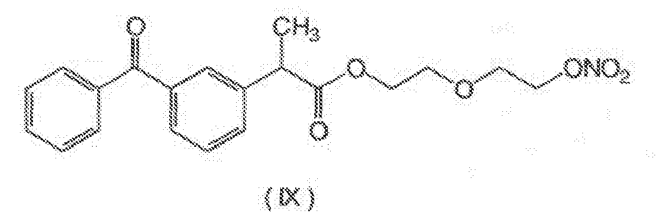
5-Benzoyl-2,3-dihydro-1H-pyrrolizin-1-carbonsäure-3-(nitrooxy)propylester 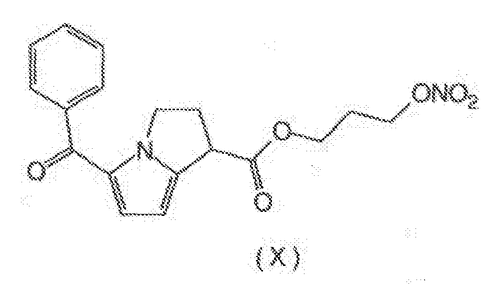
5-Benzoyl-2,3-dihydro-1H-pyrrolizin-1-carbonsäure-4-(nitrooxy)butylester 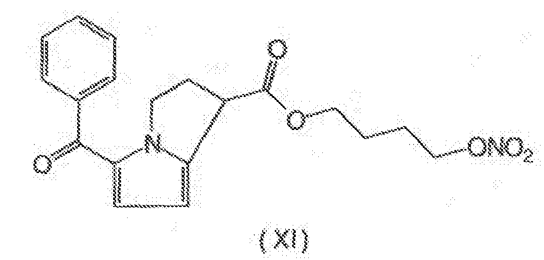
5-Benzol-2,3-dihydro-1H-pyrrolizin-1-carbonsäure-6-(nitrooxy)hexylester 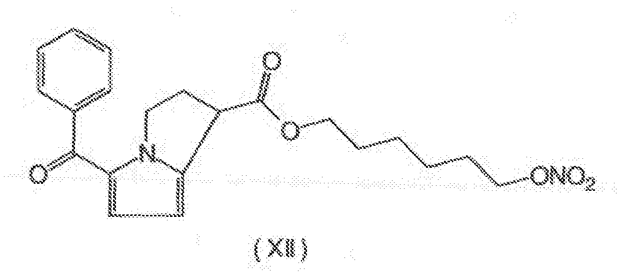
5-Benzoyl-2,3-dihydro-1H-pyrrolizin-1-carbonsäure-5-(nitrooxy)ethyloxyethylester 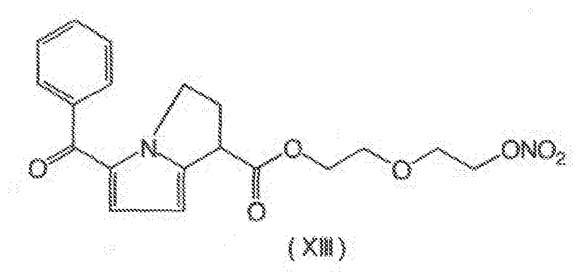
5-Benzoyl-1-2,3-dihydro-1H-pyrrolizin-1-carbonsäure-4-(nitrooxymethyl)phenylmethylester 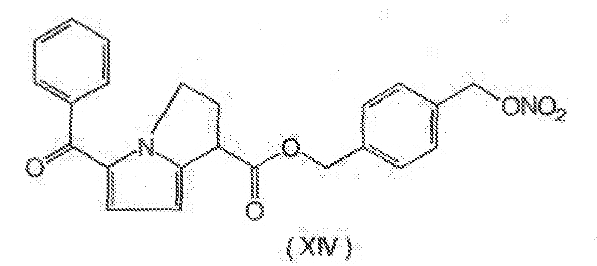
(S)-6-Methoxy-α-methyl-2-naphthalinessigsäure-6-(nitrooxy)hexylester 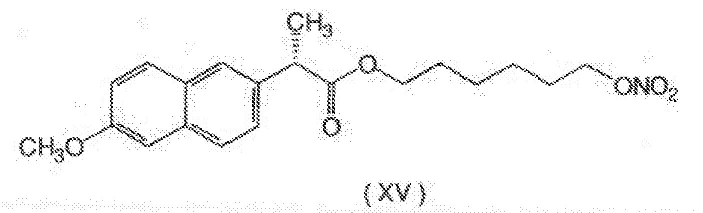
(S)-6-Methoxy-α-methyl-2-naphthalinessigsäure-5-(nitrooxy)ethyl-oxyethylester 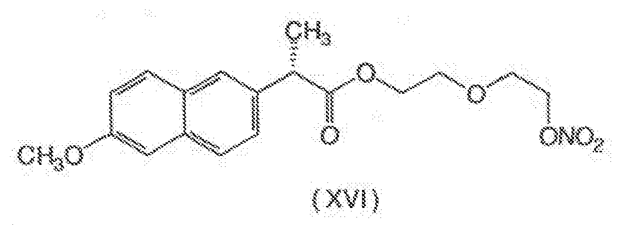
(S)-6-Methoxy-α-methyl-2-naphthalinessigsäure-4-nitrooxy-2-butenylester 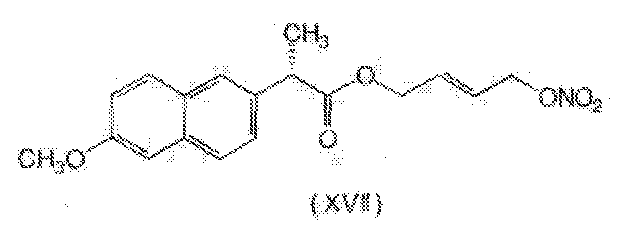
trans-3-[4-[α-Methyl-4-(2-methylpropyl)benzol]acetyloxy]-3-methoxyphenyl]-2-propensäure-4-(nitrooxy)butylester 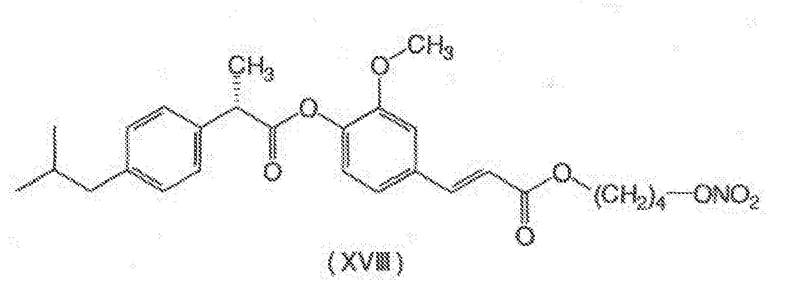
2-Fluor-α-methyl [1,1'-bipenyl]-4-essigsäure-4-(nitrooxy)butylester (NO-Flurbiprofen) 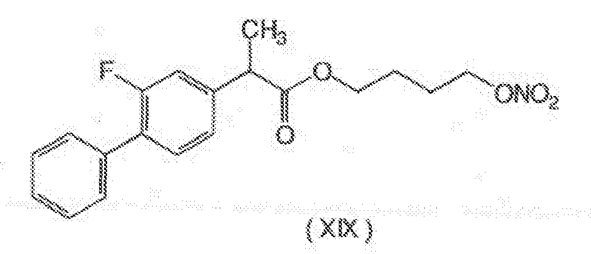
(S)-6-Methoxy-α-methyl-2-naphthalinessigsäure-4-(nitrooxy)butylester (NO-Naproxen) 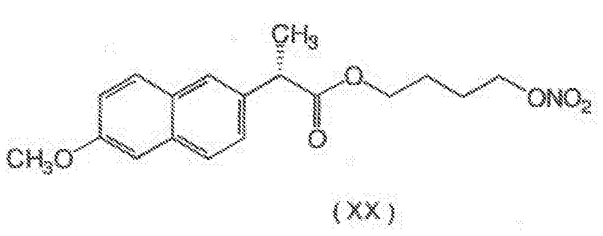
2-(Acetyloxy)benzoesäure-4-(nitrooxy)butylester 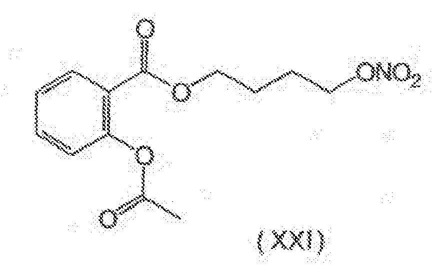
2-(Acetyloxy)benzoesäure-5-(nitrooxy)ethyloxyethylester 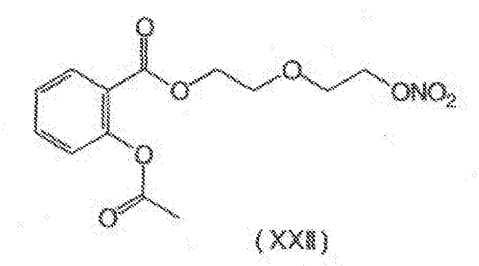
2-(6-Methoxy-α-methyl-2-naphthalinacetyl)-thiazolidine-4-carbonsäure-4-(nitrooxy)butylester 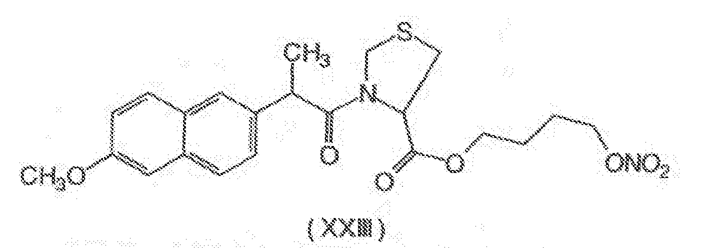
N-(2-Nitrooxyethyl)-2-fluor-α-methyl-[1,1'-biphenyl]-4-acetamid 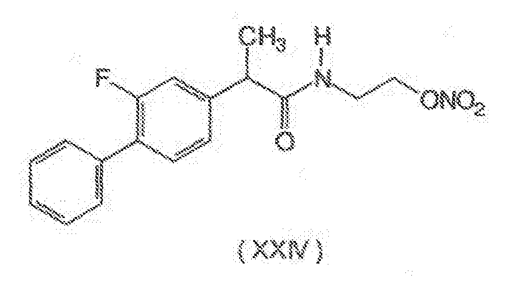
3-[2-Fluor-α-methyl-[1,1'-biphenyl]-4-acetyl]-thiazolidin-4-carbonsäure-4-(nitroxy)butylester 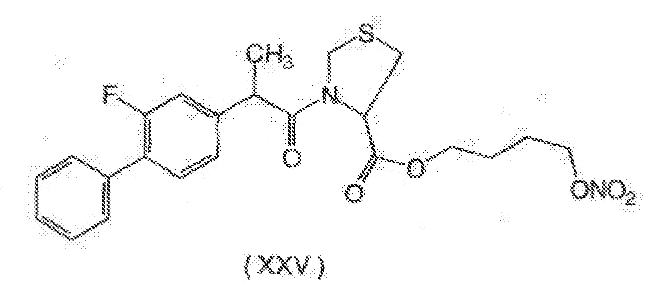
α-Methyl-4-(2-methylpropyl)benzolessigsäure-6-(nitrooxy)hexylester 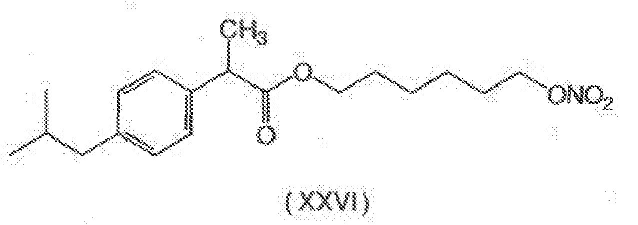
α-Methyl-4-(2-méthylpropyl)benzolessigsäure-3-(nitrooxy)propylester 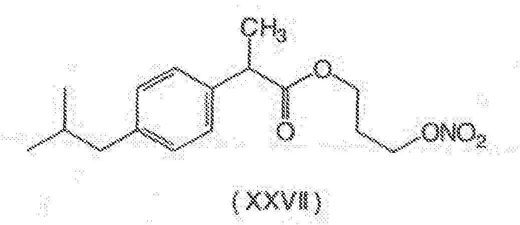
(S)-6-Methoxy-α-methyl-2-naphthalinessigsäure-1-nitrooxy-2-methyl-2-propylester 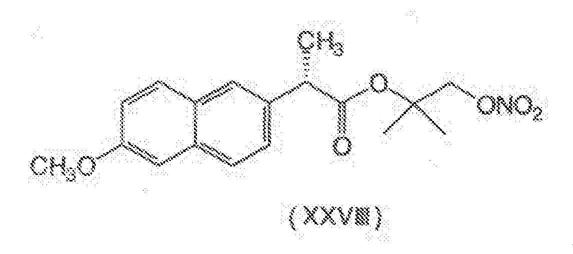
(S)-6-Methoxy-α-methyl-2-naphthalinessigsäure-10-(nitrooxy)decylester 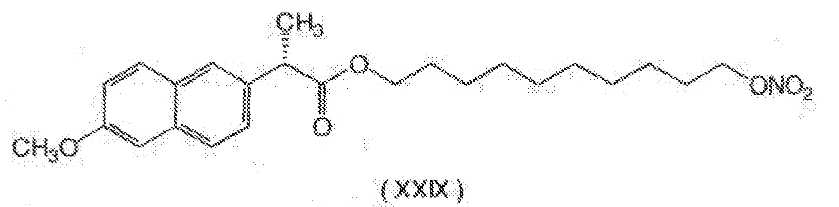
α-Metyl-4-[(2-oxocyclopentyl)methyl]benzolessigsäure-4-(nitrooxy)butylester 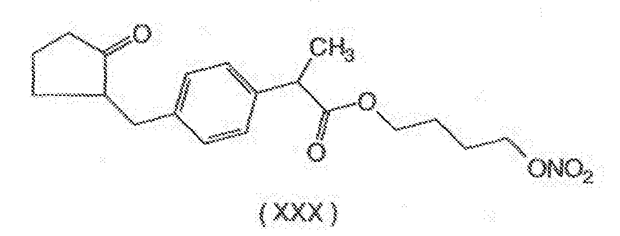
3-(6-Methoxy-α-methyl-2-naphthalinacetyl)-R(-)-2-oxothiazolidin-4-carbonsäure-4-(nitrooxy)butylester 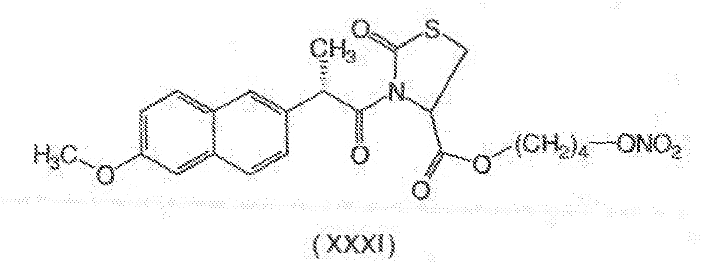
(S)-N-Acetyl-[α-methyl-4-(2-méthylpropyl)-benzolacetyl]cystein-4-(nitrooxy)butylester 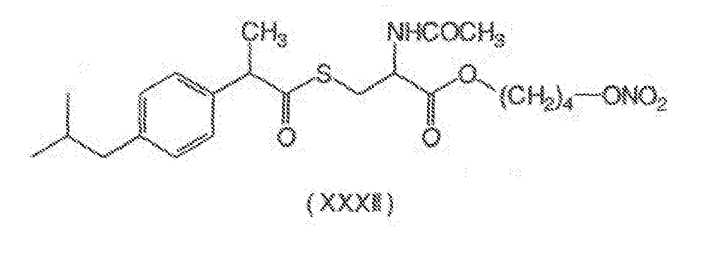
2-[(2,6-Dichlorphenyl)amino]benzolessigsäure-2-(nitrooxy)ethylester 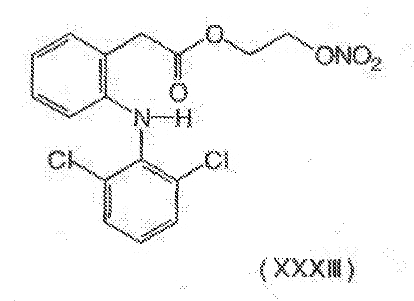
5-Benzoyl-2,3-dihydro-1H-pyrrolizin-1-carbonsäure-4-(nitrooxymethyl)phenylmethylester 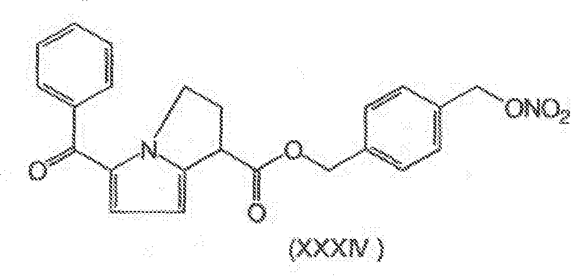
(S)-N-Acetyl-[2-fluor-α-methyl-(1,1'-biphenyl)-4-acetyl]cystein-4-(nitrooxy)butylester 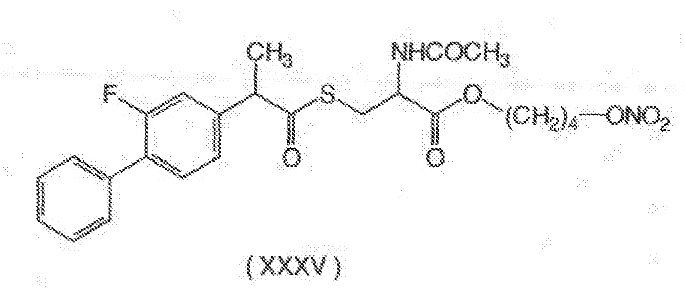
α-Methyl-4-(2-methylpropyl)benzolesigsäure-4-(nitrooxy) butylester 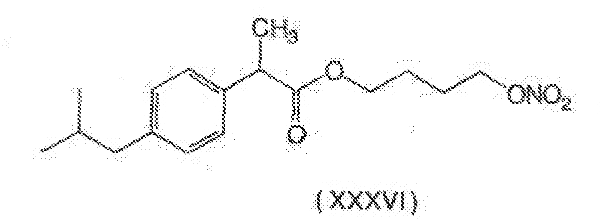
trans-3-[4-[2-Fluor-α-methyl-(1,1'-biphenyl)-4-acetyloxy]-3-methoxyphenyl]-2-propensäure-4-(nitrooxy) butylester 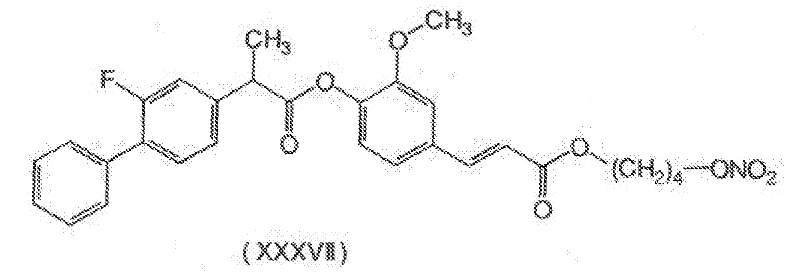
(S)-6-Methoxy-α-methyl-2-naphthalinessigsäure-4-(nitrooxy)-4-methylbutylester 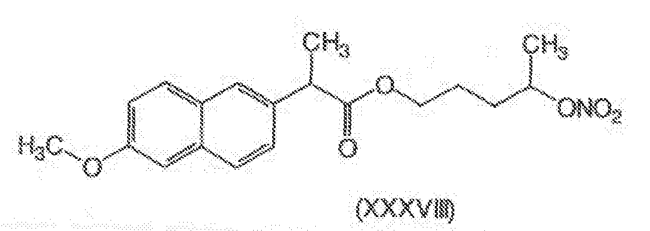
2-Fluor-α-methyl-[1,1'-biphenyl]-4-essigsäure-3-(nitrooxymethyl)phenylester 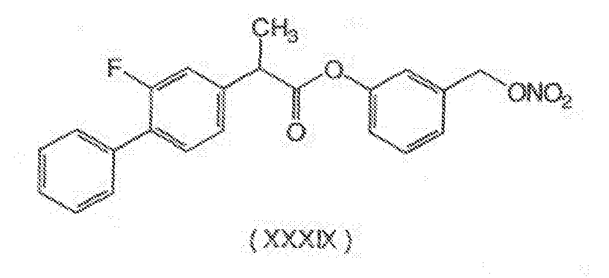
2-Fluor-α-methyl-[1,1'-biphenyl]-4-essigsäure-6-(nitrooxymethyl)-2-methylpyridylester 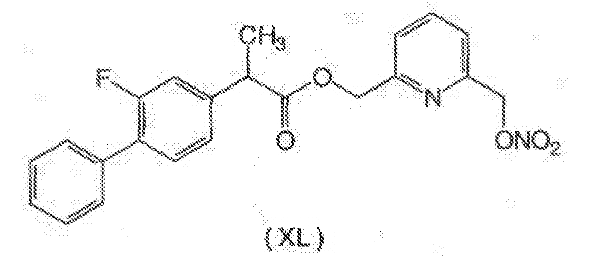
2-(Acetyloxy)benzoesäure-3-(nitrooxymethyl)-methylphenylester 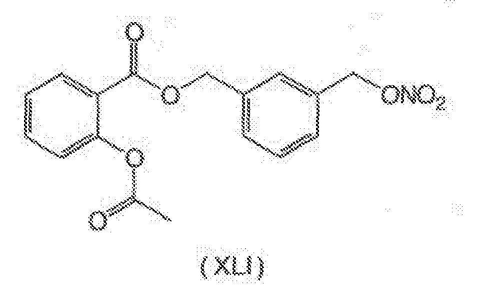
2-Fluor-α-methyl-[1,1'-biphenyl]-4-essigsäure-3-(nitrooxy)propylester

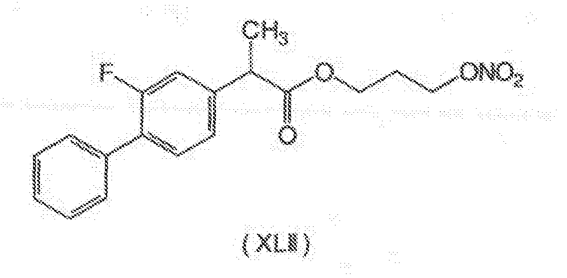

7. Pharmazeutische Zusammensetzung gemäss Ansprüchen 1 bis 4. wobei der flüssige Wirkstoff ausgewählt ist aus
4-(Nitrooxy)butansäure-2-metroimidazol-1-ethylester 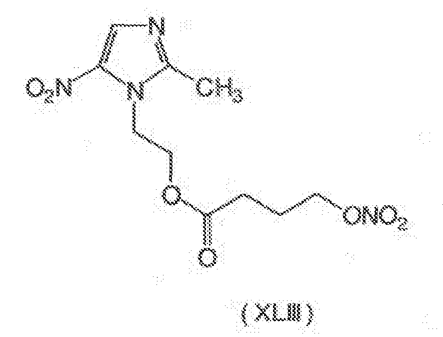
1-Ethyl-6,8-difluor-1,4-dihydro-7-(3-methyl-1-piperazinyl)-4-oxo-3-chinolincarbonsäure-4-(nitrooxy)butylester 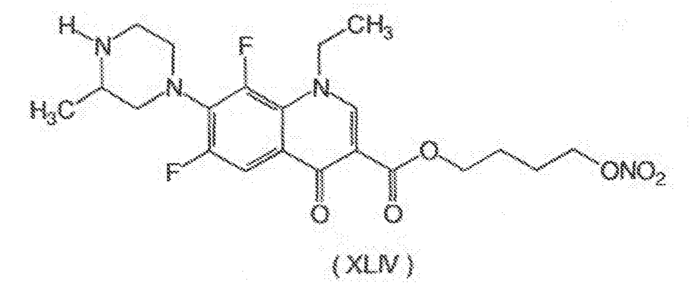
Norfloxacin-4-(nitrooxy)butylester

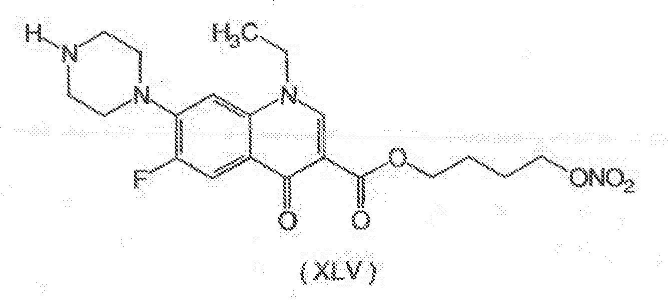

8. Pharmazeutische Zusammensetzung gemäss Ansprüchen 1 bis 4, wobei der flüssige Wirkstoff aus der Gruppe bestehend aus Nikotin, Nitroglycerin, Valproinsäure, Benzonatat, Clofibrat, Clorfeniramin, Clorfenoxamin, Clorfentermin und Clorpromazin, flüssigen Vitaminen und Mischungen hiervon ausgewählt ist.

9. Pharmazeutische Zusammensetrung gemäss Ansprüchen 1 bis 8, wobei das Tensid aus kationischen, anionischen und nicht-ionischen Tensiden, wie alkalische Seifen, organischen Aminseifen, Schwefelsäureerstern, Alkylarylsulfonat, Polyethylenglykolestern und -ethern und Polysorbaten ausgewählt ist.

10. Pharmazeutische Zusammensetzung gemäss Anspruch 9, wobei das Tensid aus der Gruppe bestehend aus Natriumstearat, Kaliumstearat, Natriumlaurylsulfat, Natriummonolaurylglycerosulfat, Benzalkoniumchlorid, Cetyltrimethylammoniumbromid, Cetrimid, Arlacel, Tween, Capmul, Cremophor, Labrafac, Labrafil und Labrasol oder Mischungen hiervon ausgewählt ist.

11. Pharmazeutische Zusammensetzung gemäss irgendeinem der vorhergehenden Ansprüche, wobei das Co-Tensid aus geradkettigen oder verzweigten Alkoholen, vorzugsweise C₁₋₆-Alkoholen, wie Ethylalkohol, Propylalkohol, Isopropylalkohol, Butylalkohol, Isobutylalkohol, und Polyolen, wie Glycerin, Ethylenglykol, Propylenglykol, Isopropylenglykol, Butylenglykol, Isobutylenglykol, ausgewählt ist.

12. Pharmazeutische Zusammensetzung gemäss irgendeinem der vorhergehenden Ansprüche, wobei der Absorptionsverstärker aus Polysorbaten, Sorbitanestern, Dioctylnatriumsulfosuccinat, Ethoxydiglykol, ethoxylierten Nonylphenolen, Polyethylenlaurylether, Phospholipidderivaten, Fettsäureestern, Gallensäurederivaten, aprotischen, Lösungsmitteln, wie Dimethylsufoxid, Dimethylformamid, Dimethylacetamid und 2-Pyrrolidon, ausgewählt ist.

13. Pharmazeutische Zusammensetzung gemäss irgendeinem der vorhergehenden Ansprüche, wobei der inerte feste Träger aus der Gruppe bestehend aus Ton, wie Bentonit, Kaolin, Silicaderivatan, wie Aerosil, Carbosil, Cellulosederivaten, wie Avicel, Silicates, wie Magnesiumtrisilicat, Talk, Erdalkalimetallhydroxiden, wie Magnesium- und Aluminiumhydroxid, Stärke, Zuckern und Cyclodextrinen ausgewählt ist.

14. Pharmazeutische Zusammensetzung gemässs Anspruch 10, wobei der inerte feste Träger Silica ist.

15. Pharmazeutische Zusammensetzung gemäss irgendeinem der vorhergehenden Ansprüche, wobei das Verhältnis von Wirkstoff : Tensid 1:0,1 bis 1:10 ist.

16. Pharmazeutische Zusammensetzung gemäss irgendeinem der vorhergehenden Ansprüche, wobei das Verhältnis von Co-Tensid : Tensid 1:0,1 bis 1:5 ist.

17. Pharmazeutische Zusammensetzung gemäss irgendeinem der vorhergehenden Ansprüche, wobei das Verhältnis von Absorptionsverstärker : Tensid 1:0,1 bis 1:10 ist.

18. Pharmazeutische Zusammensetzung gemäss irgendeinem der vorhergehenden Ansprüche, wobei das Verhältnis von Zusatzmittel: fester Träger 1:20 bis 10:1, vorzugsweise 1:2 bis 2:1, ist.

19. Pharmazeutische Zusammensetzung gemäss irgendeinem der vorhergehenden Ansprüche in Form von Tabletten, beschichteten Tabletten, Sachets und Kapseln.

## Revendications

1. Composition pharmaceutique pour administration par voie orale constituée par un véhicule inerte solide et un mélange comprenant :
i) un ou plusieurs ingrédient(s) actif(s) liquide(s)
ii) un ou plusieurs surfactant(s)
iii) éventuellement un co-surfactant et/ou
iv) éventuellement un agent d'augmentation de l'absorption,
ladite composition formant une émulsion huile dans l'eau par contact avec des milieux aqueux et étant **caractérisée en ce que** ledit mélange est absorbé dans le véhicule inerte.

2. Composition pharmaceutique selon la revendication 1, dans laquelle le mélange absorbé véhicule inerte comprend :
i) un ou plusieurs ingrédient(s) actif(s) liquide(s) ;
ii) un ou plusieurs surfactant(s) ;
iii) un agent d'augmentation de l'absorption.

3. Composition pharmaceutique selon la revendication 1 ou 2. laquelle composition forme une émulsion huile dans l'eau, avec une taille moyenne des gouttelettes de 0,05 micron à 50 microns, par contact avec des milieux aqueux tels que des fluides biologiques.

4. Composition pharmaceutique selon la revendication 1 ou 2, laquelle composition forme une émulsion huile dans l'eau, avec une taille moyenne des gouttelettes inférieure à 5 microns, par contact avec des milieux aqueux tels que des fluides biologiques.

5. Composition pharmaceutique selon les revendications 1 à 4, dans laquelle l'ingrédient actif liquide est un médicament anti-inflammatoire non stéroidien libérant NO.

6. Composition pharmaceutique selon la revendication 5, dans laquelle le médicament anti-inflammatoire non stéroïdien libérant NO est choisi dans le groupe constitué par :
l'ester 3-(nitrooxy)propylique de l'acide (S)-3-benzoyl-α-méthylbenzèneacétique 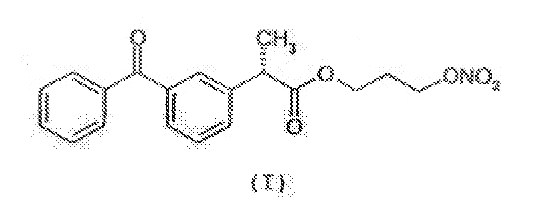
l'ester 4-(nitrooxyméthyl)phényméthylique de l'acide (S)-3-benzoyl-α-méthylbenzèneacétique 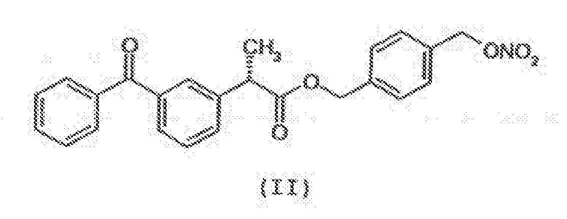
l'ester 5-(nitrooxy)éthyloxyéthylique de l'acide 2-[(2,6-dichlorophényl)amino]benzèneacétique 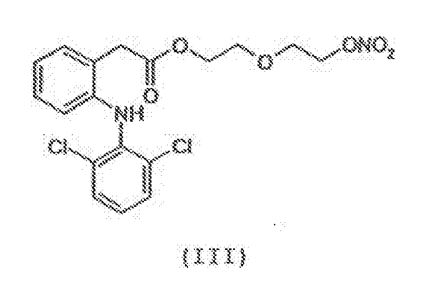
l'ester 4-(nitrooxy)butylique de l'acide 2-[(2,6-diclorophényl)amino]benzèneacétique (NO-Diclofénac) 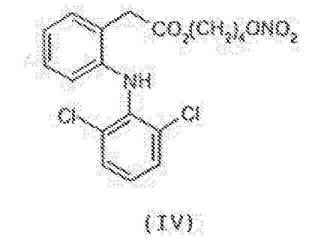
l'ester 3-(nitrooxy)propylique de l'acide 2-[(2,6-dichlorophényl)amino]benzèneacétique 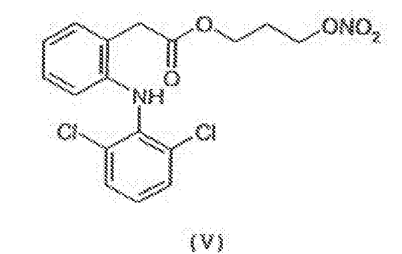
l'ester 6-(nitrooxy)hexylique de l'acide 2-[(2,6-dichlorophényl)amino]benzèneacétique 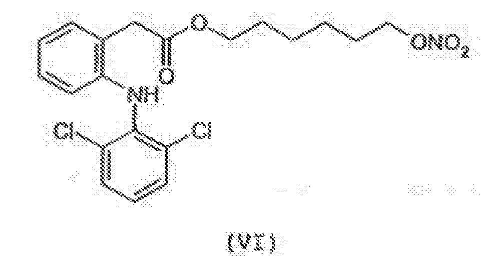
l'ester 4-(nitrooxy)butylique de l'acide 3-benzoyl-α-méthylbenzèneacétique 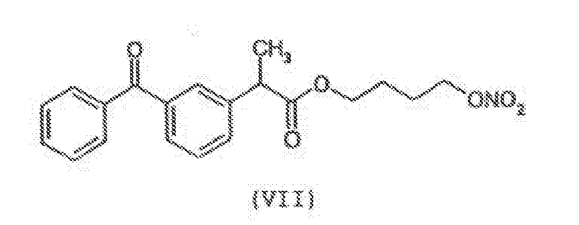
l'ester 6-(nitrooxy)hexylique de l'acide 3-benzoyl-α-méthylbenzèneacétique 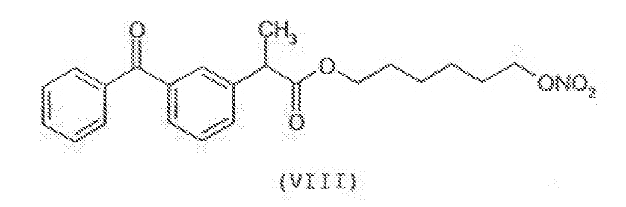
l'ester 5-(nitrooxy)éthyloxyéthylique de l'acide 3-benzoyl-α-méthylbenzèneacétique 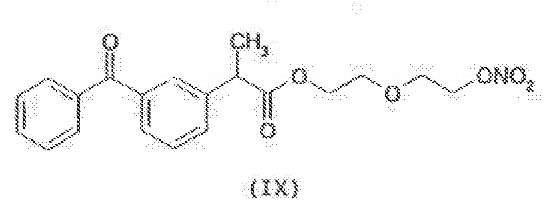
l'ester 3-(nitrooxy)propylique de l'acide 5-benzoyl-2,3-dihydro-1H-pyrrolizin-1-carboxylique 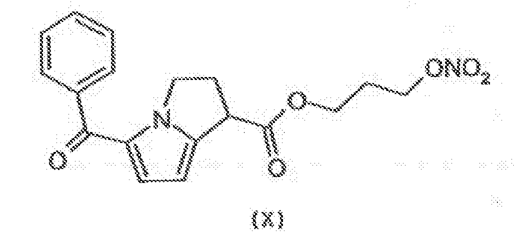
l'ester 4-(nitrooxy)butylique de l'acide 5-benzoyl-2,3-dihydro-1H-pyrrolizin-1-carboxylique 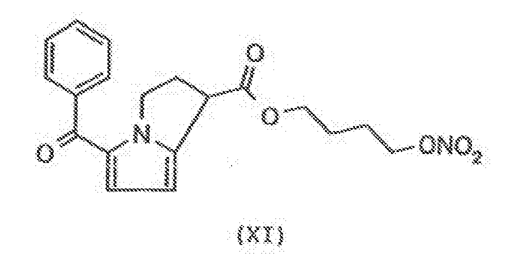
l'ester 6-(nitrooxy)hexylique de de l'acide 5-benzoyl-2,3-dihydro-1H-pyrrolizin-1-carboxylique 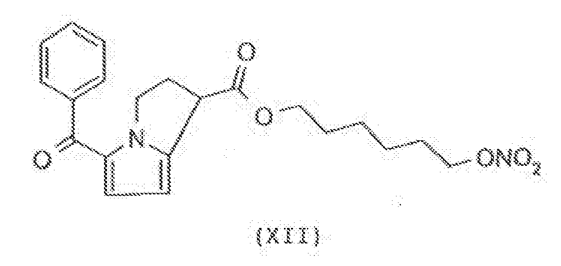
l'ester 5-(nitrooxy)éthyloxyéthylique de l'acide 5-benzoyl-2,3-dihydro-1H-pyrrolizin-1-carboxylique 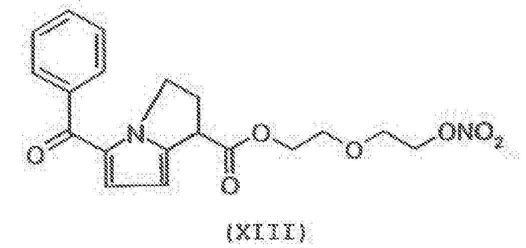
l'ester 4-(nitrooxyméthyl)phénylméthylique de l'acide 5-benzoyl-2,3-dihydro-1H-pyrrolizin-1-carboxylique 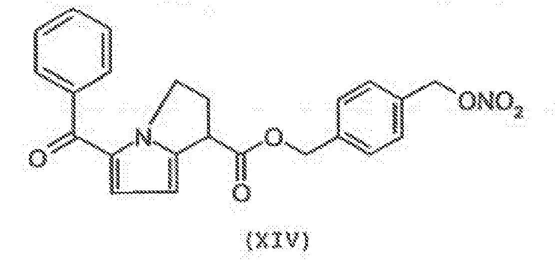
l'ester 6-(nitroxy)hexylique de l'acide (S)-6-méthoxy-α-méthyl-2-naphtalèneacétique 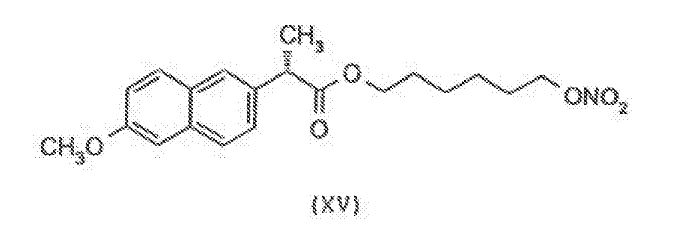
l' ester 5 (nitrooxy)éthyloxyéthylique de l' acide (S)-6-methoxy-α-méthyl-2-naphtalèneacétique 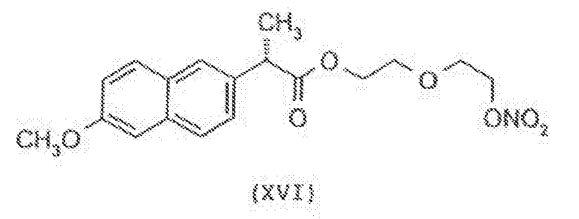
l'ester 4-nitrooxy-2-buténylique de l'acide (S)-6-méthoxy-α-méthyl-2-naphtalèneacétique 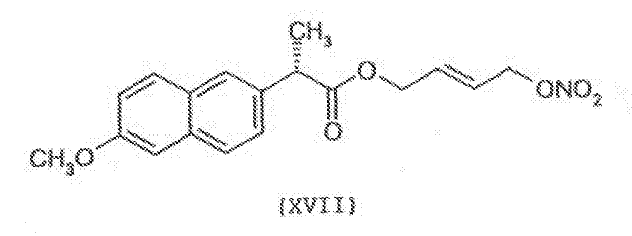
l'ester 4-(nitrooxy)butylique de l'acide trans-3-[4-[α-méthyl-4-(2-méthylpropyl)benzène]acétyloxy-3-méthoxyphényl]-2-propénoïque 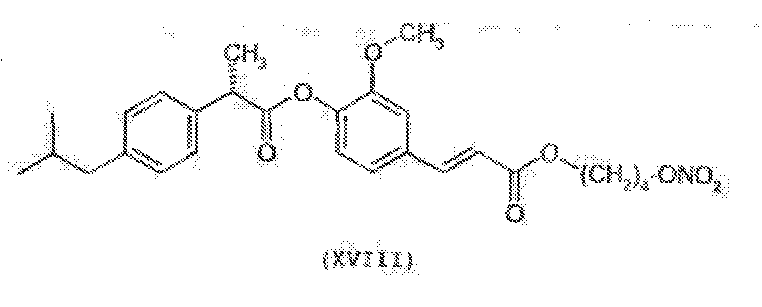
l'ester 4-(nitrooxy)butylique de l'acide 2-fluoro-α-méthyl-[1,1'-biphényl]-4-acétique (NO-Fluorbiprofène) 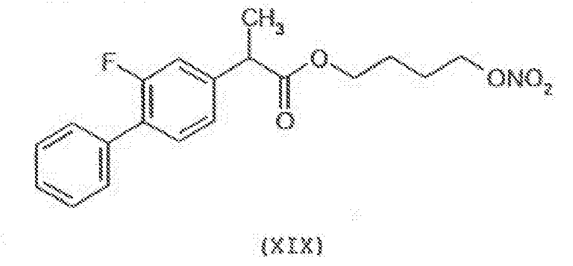
l'ester 4-(nitrooxyl)butylique de l'acide (S)-6-méthoxy-α-méthyl-2-naphtalèneacétique (NO-Naproxène) 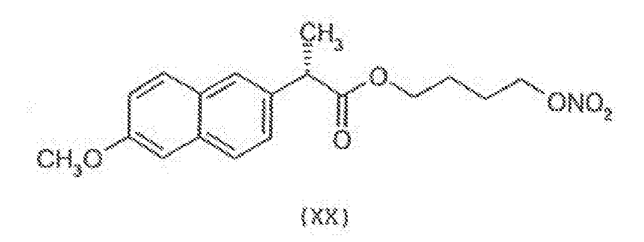
l'ester 4-(nitrooxy)butylique de l'acide 2-(acétyloxy)benzoïque 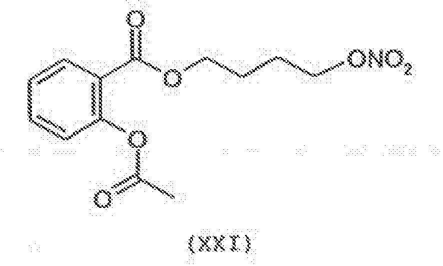
l'ester 5-(nitrooxy)éthyloxyéthylique de l'acide 2-(acétyloxy)benzoïque 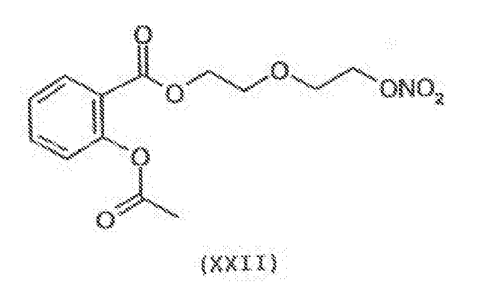
l'ester 4-(nitrooxy)butylique de l'acide 3-(6-méthoxy-α-méthyl-2-naphtalèneacétyl)thiazolidin-4-carboxylique 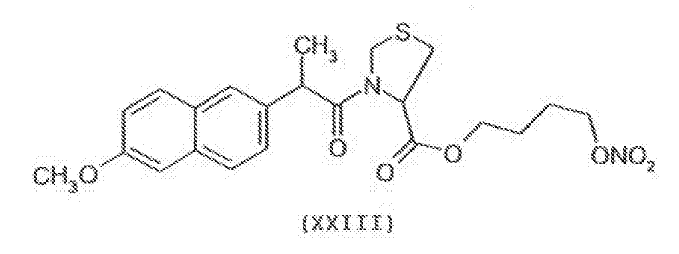
le N-(2-nitrooxyéthyl)-2-fluoro-α-méthyl)-[1,1'-biphényl]-4-acétamide 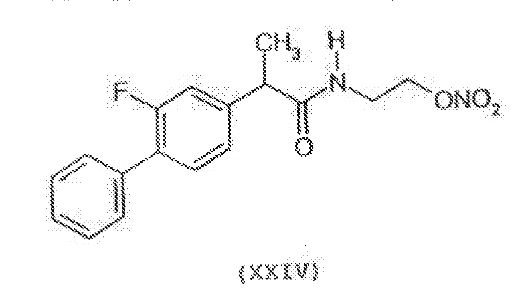
l'ester 4-(nitrooxy)butylique de l'acide 3-[2-fluoro-α-méthyl-[1,1'-biphényl]-4-acétyl]thiazolidin-4-carboxylique 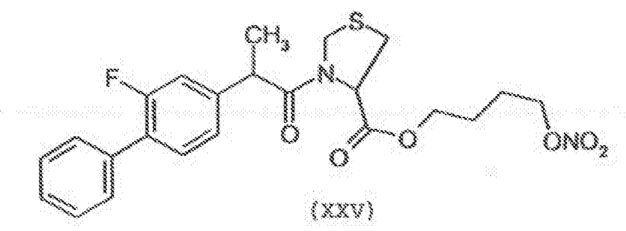
l'ester 6-(nitrooxy)hexylique de l'acide α-méthyl-4-(2-méthylpropyl)benzèneacétique 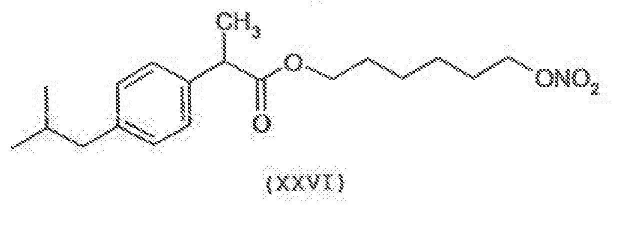
l'ester 3-(nitrooxy)propylique de l'acide α-méthyl-4-(4-méthylpropyl)benzèneacétique 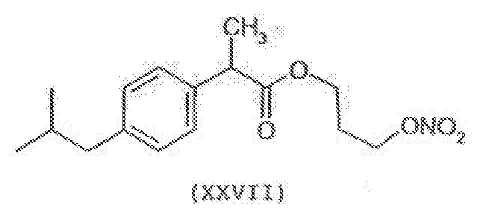
l'ester 1-nitrooxy-2-méthyl-2-propylique de l'acide (S)-6-méthoxy-α-méthyl-2-naphtalèneacétique 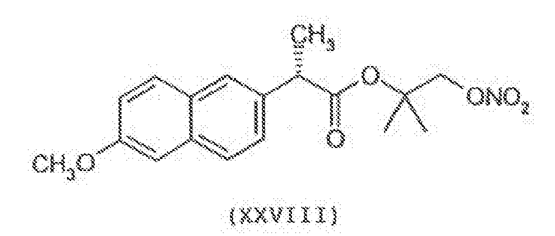
l'ester 10-(nitrooxy)décylique de l'acide (S)-6-méthoxy-α-méthyl-2-naphtalèneacétique 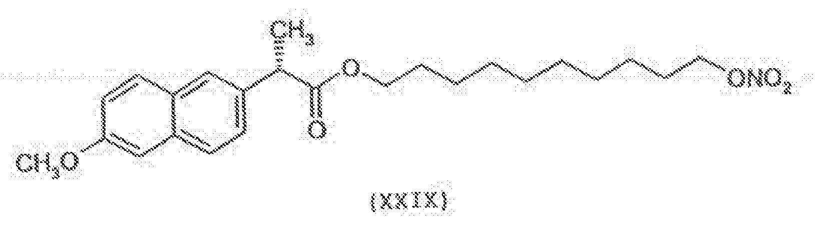
l'ester 4-(nitrooxy)butylique de l'acide α-méthyl-4-[(2-oxocyclopentyl)méthyl]benzèneacétique 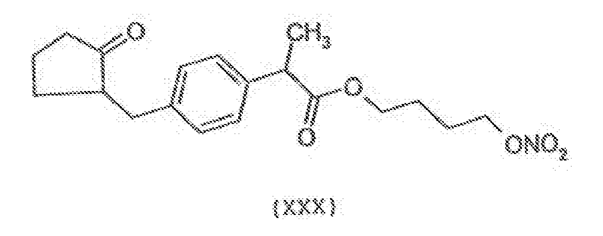
l'ester 4-(nitrooxy)butylique de l'acide 3-(6-méthoxy-α-méthyl-2-napthalèneacétyl)-R(-)-2-oxothiazolidin-4-carboxylique 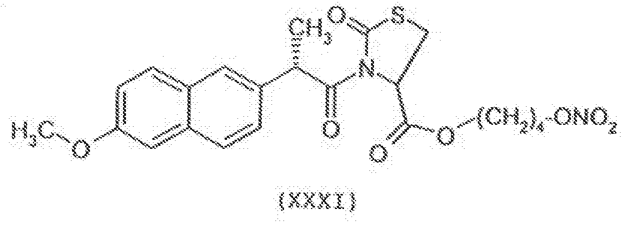
l'ester 4-(nitrooxy)butylique de la (S)-N-acétyl-[α-méthyl-4-(2-méthylpropyl)benzèneacétyl]cystéine 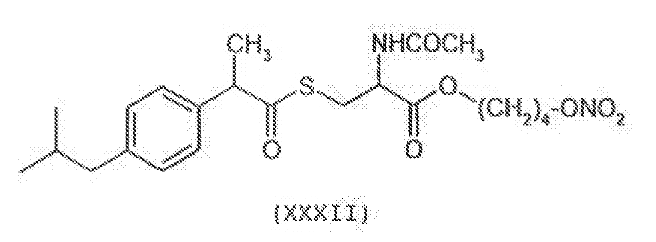
l'ester 2-(nitrooxy)éthylique de l'acide 2-[(2,6-dichlorophényl)amino]benzèneacétique 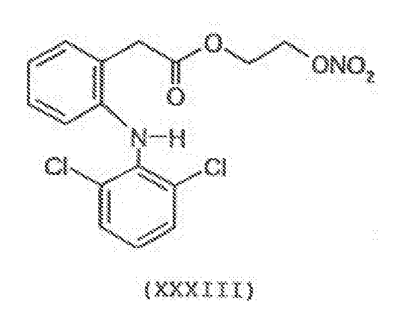
l'ester 4-(nitrooxyméthyl)phénylméthylique de l'acide 5-benzoyl-2,3-dihydro-1H-pyrrolizin-1-carboxylique 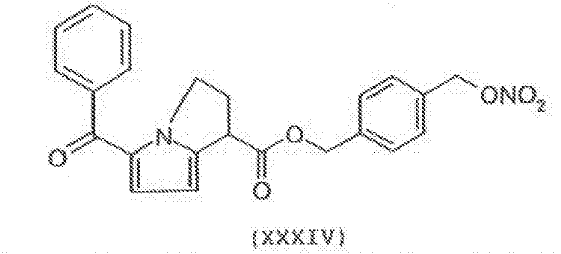
l'ester 4-(nitrooxy)butylique de la (S) -N-acétyl-[2-fluoro-α-méthyl-(1,1'-biphényl)-4-acétyl]cystéine 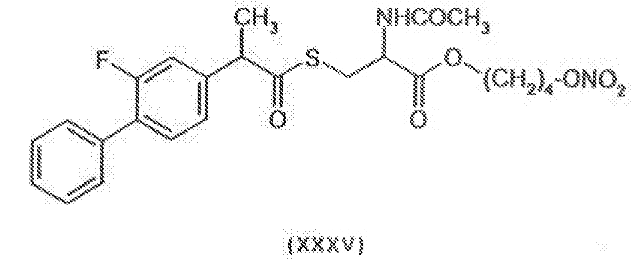
l'ester 4-(nitrooxy)butylique de l'acide α-méthyl-4-(2-méthylpropyl)benzèneacétique 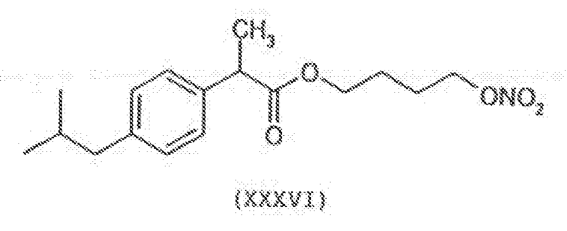
l'ester 4-(nitrooxy)butylique de l'acide trans-3-[4-[2-fluoro-α-méthyl-(1,1'-biphényl)-4-acétyloxyl-3-méthoxyphényl]-2-propénoïque 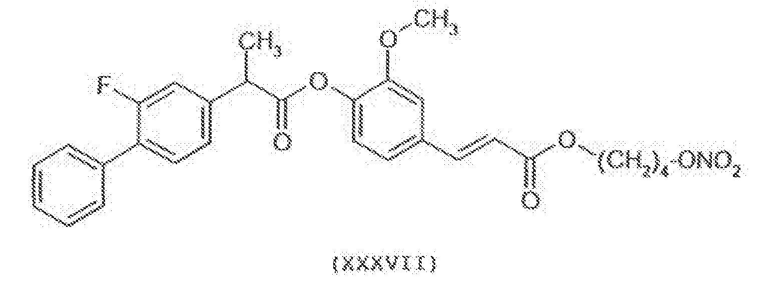
l'ester 4-(nitrooxy)-4-méthylbutylique de l'acide (S)-6-méthoxy-α-méthyl-2-naphtalèneacétique 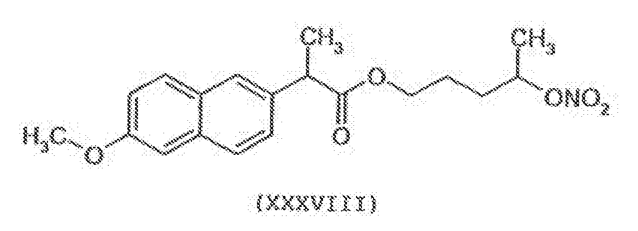
l'ester 3-(nitrooxyméthyl)phénylique de l'acide 2-fluoro-α-méthyl-[1,1'-biphényl]-4-acétique 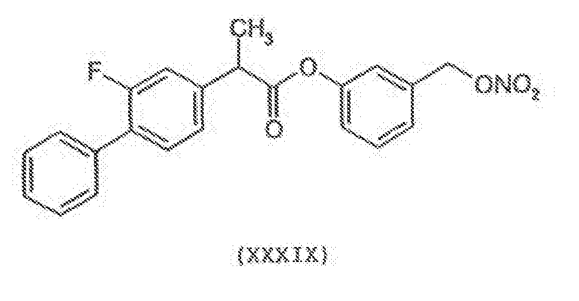
l'ester 6-(nitrooxyméthyl)-2-méthylpyridylique de l'acide 2-fluoro-α-méthyl-[1,1'-biphényl]-4-acétique 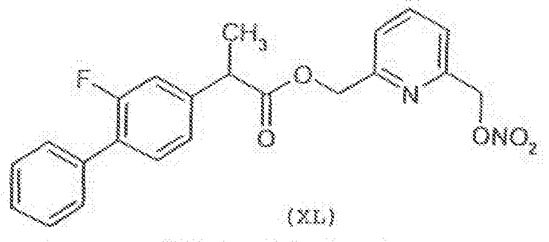
l'ester 3-(nitrooxyméthyl)méthylphénylique de l'acide 2-(acétyloxy)benzoïque. 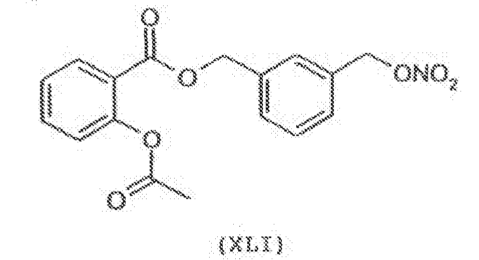
l'ester 3-(nitrooxy)propylique de l'acide 2-fluoro-α-méthyl-[1,1'-biphényl]-4-acétique

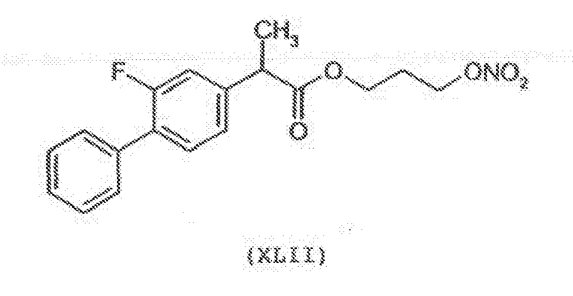

7. Composition pharmaceutique selon les revendications 1 à 4, dans laquelle l'ingrédient actif liquide est choisi parmi l'ester 2-méthyl-5-nitroimidazole-1-éthylique de l'acide 4-(nitrooxy)butanoïque 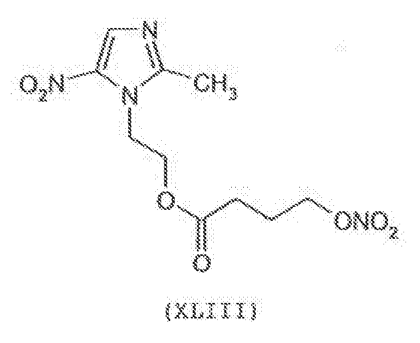
l'ester 4-(nitrooxy)butylique de l'acide l'éthyl-6,8-difluoro-1,4-dihydro-7-(3-méthyl-1-pipérazinyl)-4-oxo-3-quinoléinecarboxylique 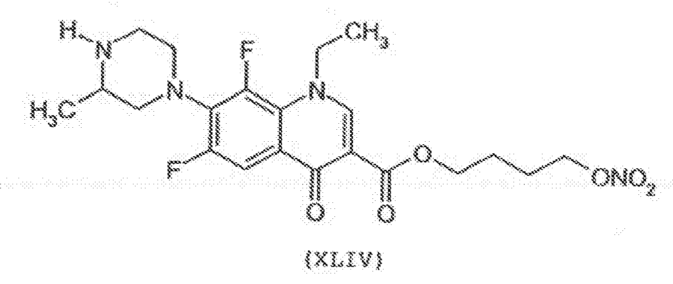
l'ester 4-(nitrooxy)butylique de la norfloxacine

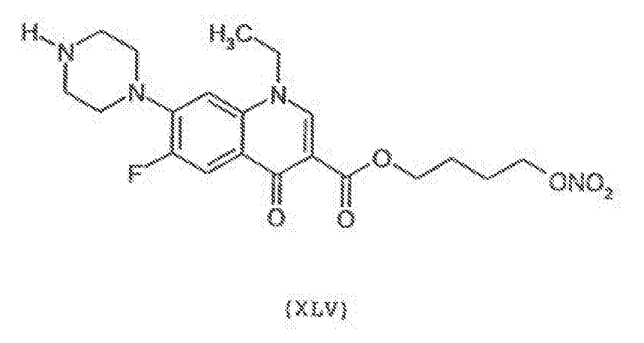

8. Composition pharmaceutique selon les revendications 1-4, dans laquelle l'ingrédient actif liquide est choisi dans le groupe constitué par la nicotine, la nitroglycérine, l'acide valproïque, le benzonatate, le clofibrate, la clorféniramine, la clorfénoxamine, la clorfentermine et la clorpromazine, les vitamines liquides et les mélanges de ceux-ci.

9. Composition pharmaceutique selon les revendications 1-8, dans laquelle le surfactant est choisi parmi les surfactants cationiques, anioniques et non ioniques tels que des savons alcalins, des savons d'amines organiques, des esters sulfuriques, des alkylarylsulfonates, des esters et éthers de polyéthylène glycol, des polysorbates.

10. Composition pharmaceutique selon la revendication 9, dans laquelle le surfactant est choisi dans le groupe constitué par le stéarate de sodium, le stéarate de potassium, le laurylsulfate de sodium, le monolauryl glycérosulfate de sodium, le chlorure de benzalkonium, le bromure de cétyltriméthylammonium, le cétrimide, l'Arlacel, le Tween, le Capmul, le Crémophor, le Labrafac, le Labrafil et le Labrasol et des mélanges de ceux-ci.

11. Composition pharmaceutique selon l'une quelconque des revendications précédentes, dans laquelle le co-surfactant est choisi parmi les alcools à chaîne linéaire ou ramifiée, de préférence des alcools en C₁-C₆, tels que l'alcool éthylique, l'alcool propylique, l'alcool isopropylique, l'alcool butylique, l'alcool isobutylique, et les polyols tels que le glycérol, l'éthylène glycol, le propylène glycol, l'isopropylène, glycol, le butylène glycol, l'isobutylène glycol,

12. Composition pharmaceutique selon l'une quelconque des revendications précédentes, dans laquelle l'argent d'augmentation de l'absorption est choisi parmi les polysorbates, les esters de sorbitan, le dioctyl sulfosuccinate de sodium, l'éthoxydiglycol, les nonylphénols éthoxylés, le polyéthylène lauryléther, les dérivée phospholipidiques, les esters d'acides gras, les dérivée d'acides biliaires, les solvants aprotiques tels que le diméthylsulfoxyde, le diméthylformamide, le diméthylacétamide et la 2-pyrrolidone.

13. Composition pharmaceutique selon l'une quelconque des revendications précédentes, dans laquelle le véhicule solide inerte est choisi dans le groupe constitué par les argiles telles que la bentonite, le kaolin, des dérivés de la silice tels que l'Aérosil, le Carbosil, des dérives de la cellulose tels que l'Avicel, les silicates tels que le trisilicate de magnésium, le talc, les hydroxydes de métaux alcalino-terreux tels que les hydroxydes d'aluminium et de magnésium, l'amidon, les sucres et les cyclodextrines.

14. Composition pharmaceutique selon la revendication 10, dans laquelle le véhicule solide inerte est la silice.

15. Composition pharmaceutique selon l'une quelconque des revendications précédentes, dans laquelle le rapport ingrédient actif : surfactant est de 1: 0,1 à 1 : 10.

16. Composition pharmaceutique selon l'une quelconque des revendications précédentes, dans laquelle le rapport co-surfactant : surfactant est de 1 : 0,1 à 1 : 5.

17. Composition pharmaceutique selon l'une quelconque des revendications précédentes, dans laquelle le rapport agent d'augmentation de l'absorption : surfactant est de 1 : 0,1 à 1 : 10.

18. Composition pharmaceutique selon l'une quelconque des revendications précédentes, dans laquelle le rapport mélange : véhicule solide est de 1 : 20 à 10 : 1, de préférence de 1 : 2 à 2 : 1.

19. Composition pharmaceutique selon l'une quelconque des revendications précédentes sous la forme de comprimés, de comprimés revêtus, de sachets et de capsules.
